# EUROPEAN PATENT APPLICATION

(11) **EP 4 825 375 A1**
(43) Date of publication of application: **21.10.2026**
(21) Application number: 24902975.2
(22) Date of filing: 13.12.2024
(51) Int. Cl.: A61K 9/51, A61K 9/127, A61K 47/18, A61K 31/7088, A61K 31/7105, A61P 25/28, A61P 29/00, A61P 31/00, A61P 35/00, A61P 37/00, A61P 37/02, C07C 217/08, C07C 219/16, C07C 229/12, C07C 229/16

(54) **COMPOSITION AND COMPOUND FOR DELIVERING THERAPEUTIC AGENT**

(30) Priority: 13.12.2023 CN 202311714690
(71) Applicant: Shanghai Regenelead Therapies Co., Ltd., Shanghai 201206 (CN)
(72) Inventor: LIU, Chongyi, Shanghai 201206 (CN); JIANG, Jun, Shanghai 201206 (CN); HUANG, Jian, Shanghai 201206 (CN); ZHU, Lingjian, Shanghai 201206 (CN); KUANG, Jingwen, Shanghai 201206 (CN); ZOU, Yang, Shanghai 201206 (CN); NING, Wei, Shanghai 201206 (CN); LIAO, Cheng, Shanghai 201206 (CN)
(74) Representative: Dragotti & Associati S.P.A.
(86) International application number: PCT/CN2024/139230
(87) International publication number: WO 2025/124560

(57) **Abstract**

A lipid nanoparticle composition for delivering a therapeutic agent, comprising a compound of formula I or compounds of formula I and formula II, or a salt thereof.

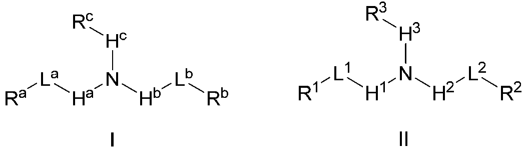

## Description

### FIELD OF THE INVENTION

The present disclosure belongs to the field of medicine, and relates to a composition and compound for delivering a therapeutic agent.

### BACKGROUND OF THE INVENTION

Nucleic acid-based drugs, such as messenger RNAs (mRNA), antisense oligonucleotides, small interfering RNAs (siRNA), plasmids, have a wide application prospect. How to safely and effectively deliver the drugs to target organs and cells within the body is a problem that hinders the development of this technology.

At present, the delivery systems for nucleic acid drugs can be divided into viral vector systems and non-viral systems. Lipid nanoparticle-mediated delivery for nucleic acid drugs is the main way of the non-viral delivery systems.

In gene therapy and vaccine applications, lipid nanoparticles (LNP) have been identified to be excellent carriers for nucleic acids for treating various diseases. Lipid nanoparticles formed from cationic lipids and other helper lipids such as cholesterol, phospholipids and PEGylated lipids can encapsulate nucleic acids, protecting them from degradation and promoting cellular uptake, thus reducing immune responses. In addition, intracellular delivery of bioactive components via lipid nanoparticles has other advantages, such as good targeting, small side effect, good stability, and high transfection efficiency.

Historically, an effective LNP is composed of four components, an ionizable cationic lipid, a zwitterionic phospholipid, cholesterol, and lipid-poly(ethylene glycol) (PEG). However, these LNPs only result in general delivery of nucleic acids, rather than delivery to target organs or tissues. LNP typically only delivers RNA to liver. CN112930198A discloses a new class of LNPs prepared by adding DOTAP (permanent cation) in addition to 5A2-SC8 (ionizable cation), DOPE (zwitter ion), cholesterol, and DMG-PEG, to achieve targeting spleen and lung. This application has demonstrated the specificity of DOTAP, since the corresponding effect could not be achieved upon replacement with other lipids.

The rapid development in the field of nucleic acid molecule-based therapy has generated an increased demand for the delivery of nucleic acid drugs. Therefore, there is a need to develop efficient and safe delivery carriers for nucleic acids.

Lipid compositions for delivering nucleic acid drugs that have already been on the market at present can only target liver tissue or be administrated topically. Therefore, the development of new delivery carriers for nucleic acid drugs enabling targeted delivery to other organs is of great significance to expand application of nucleic acid drugs.

### SUMMARY OF THE INVENTION

The present disclosure provides a lipid nanoparticle composition comprising a compound of formula I or a salt thereof, 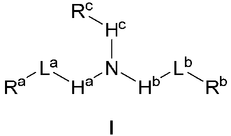 wherein,
L^{a} and L^{b} are each independently selected from the group consisting of -O-, -C(O)-, -OC(O)-, -C(O)O-, -OC(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
H^{a}, H^{b} and H^{c} are each independently selected from the group consisting of C₁₋₁₀ alkylene;
R^{a} and R^{b} are each independently selected from the group consisting of C₁₋₂₄ alkyl and C₂₋₂₄ alkenyl;
R^{c} is selected from the group consisting of NR^{c1}R^{c2} and N⁺R^{c3}R^{c4}R^{c5};
R^{c1} and R^{c2} are each independently selected from the group consisting of hydrogen and C₁₋₆ alkyl;
R^{c3}, R^{c4} and R^{c5} are each independently selected from the group consisting of C₁₋₆ alkyl.

In some embodiments, only the following substituents or groups are defined, and the definitions of substituents or groups not mentioned are the same as those described in any one of other embodiments (hereinafter referred to as "in some embodiments"), such that L^{a} and L^{b} are each independently selected from the group consisting of -OC(O)-, -C(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-.

In some embodiments, at least one (for example one) of L^{a} and L^{b} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-.

In some embodiments, at least one (for example one) of L^{a} and L^{b} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, wherein C₁₋₆ alkylene is not n-propylidene.

In some embodiments, L^{a} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and L^{b} is selected from the group consisting of -OC(O)- and -C(O)O-.

In some embodiments, L^{b} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and L^{a} is selected from the group consisting of -OC(O)- and -C(O)O-.

In some embodiments, L^{a} and L^{b} are each independently selected from the group consisting of -OC(O)-, -C(O)O-, -OCH₂C(O)O-, -OC(O)CH₂O-, -OCH₂OC(O)-, -C(O)OCH₂O-, -O(CH₂)₂OC(O)- and -C(O)O(CH₂)₂O-.

In some embodiments, L^{a} and L^{b} are each independently selected from the group consisting of -OC(O)-, -C(O)O-, -OCH₂C(O)O-, -OC(O)CH₂O-, -OCH₂OC(O)-, -C(O)OCH₂O-, -O(CH₂)₂OC(O)- and -C(O)O(CH₂)₂O-, and at least one (for example one) of L^{a} and L^{b} is selected from the group consisting of -OCH₂C(O)O-, -OC(O)CH₂O-, -OCH₂OC(O)-, -C(O)OCH₂O-, -O(CH₂)₂OC(O)- and -C(O)O(CH₂)₂O-.

In some embodiments, L^{a} is selected from the group consisting of -OCH₂C(O)O-, -OC(O)CH₂O-, -OCH₂OC(O)-, -C(O)OCH₂O-, -O(CH₂)₂OC(O)- and -C(O)O(CH₂)₂O-, and L^{b} is selected from the group consisting of -OC(O)- and -C(O)O-.

In some embodiments, L^{b} is selected from the group consisting of -OCH₂C(O)O-, -OC(O)CH₂O-, -OCH₂OC(O)-, -C(O)OCH₂O-, -O(CH₂)₂OC(O)- and -C(O)O(CH₂)₂O-, and L^{a} is selected from the group consisting of -OC(O)- and -C(O)O-.

In some embodiments, in L^{a} and L^{b}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of straight or branched C₁₋₆ alkylene.

In some embodiments, in L^{a} and L^{b}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of C₁ alkylene, C₂ alkylene, C₃ alkylene, C₄ alkylene, C₅ alkylene and C₆ alkylene.

In some embodiments, in L^{a} and L^{b}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of C₁₋₄ alkylene.

In some embodiments, in L^{a} and L^{b}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of methylene, ethylidene, n-propylidene, iso-propylidene, n-butylidene, sec-butylidene, iso-butylidene and tert-butylidene.

In some embodiments, in L^{a} and L^{b}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of methylene and ethylidene.

In some embodiments, in L^{a} and L^{b}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is not n-propylidene.

In some embodiments, H^{a} is selected from the group consisting of straight or branched C₁₋₁₀ alkylene. In some embodiments, H^{a} is selected from the group consisting of straight or branched C₃₋₈ alkylene. In some embodiments, H^{a} is selected from the group consisting of -(CH₂)₃-, -(CH₂)₄-, -(CH₂)₅-, -(CH₂)₆-, -(CH₂)₇- and -(CH₂)₈-.

In some embodiments, H^{b} is selected from the group consisting of straight or branched C₁₋₁₀ alkylene. In some embodiments, H^{b} is selected from the group consisting of straight or branched C₃₋₈ alkylene. In some embodiments, H^{b} is selected from the group consisting of -(CH₂)₃-, -(CH₂)₄-, -(CH₂)₅-, -(CH₂)₆-, -(CH₂)₇- and -(CH₂)₈-.

In some embodiments, H^{c} is selected from the group consisting of straight or branched C₁₋₁₀ alkylene. In some embodiments, H^{c} is selected from the group consisting of straight or branched C₁₋₆ alkylene. In some embodiments, H^{c} is selected from the group consisting of straight or branched C₁₋₄ alkylene. In some embodiments, H^{c} is selected from the group consisting of -CH₂-, -(CH₂)₂-, -(CH₂)₃- and -(CH₂)₄-.

In some embodiments, in H^{a}, H^{b} and H^{c}, the C₁₋₁₀ alkylene is each independently straight C₁₋₁₀ alkylene.

In some embodiments, in H^{a}, H^{b} and H^{c}, the C₁₋₁₀ alkylene is each independently branched C₁₋₁₀ alkylene.

In some embodiments, in H^{a}, H^{b} and H^{c}, the C₁₋₁₀ alkylene is each independently selected from the group consisting of C₁ alkylene, C₂ alkylene, C₃ alkylene, C₄ alkylene, C₅ alkylene, C₆ alkylene, C₇ alkylene, C₈ alkylene, C₉ alkylene and C₁₀ alkylene.

In some embodiments, in H^{a}, H^{b} and H^{c}, the C₁₋₁₀ alkylene is each independently selected from the group consisting of C₃₋₈ alkylene.

In some embodiments, in H^{a}, H^{b} and H^{c}, the C₁₋₁₀ alkylene is each independently selected from the group consisting of C₁₋₆ alkylene. In some embodiments, in H^{a}, H^{b} and H^{c}, the C₁₋₁₀ alkylene is each independently selected from the group consisting of C₁₋₄ alkylene. In some embodiments, in H^{a}, H^{b} and H^{c}, the C₁₋₁₀ alkylene is each independently selected from the group consisting of methylene, ethylidene, n-propylidene, iso-propylidene, n-butylidene, sec-butylidene, iso-butylidene and tert-butylidene.

In some embodiments, R^{a} and R^{b} are each independently selected from the group consisting of straight or branched C₁₋₂₄ alkyl.

In some embodiments, R^{a} and R^{b} are each independently selected from the group consisting of straight or branched C₄-₁₈ alkyl.

In some embodiments, R^{a} is selected from the group consisting of straight C₄-₁₈ alkyl, and R^{b} is selected from the group consisting of branched C₄-₁₈ alkyl.

In some embodiments, R^{a} is selected from the group consisting of branched C₄-₁₈ alkyl, and R^{b} is selected from the group consisting of branched C₄-₁₈ alkyl.

In some embodiments, R^{a} is selected from the group consisting of branched C₄-₁₈ alkyl, and R^{b} is selected from the group consisting of straight C₄-₁₈ alkyl.

In some embodiments, in R^{a} and R^{b}, the C₁₋₂₄ alkyl is each independently selected from the group consisting of C₁ alkyl, C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, C₂₃ alkyl and C₂₄ alkyl. In some embodiments, in R^{a} and R^{b}, the C₁₋₂₄ alkyl is each independently selected from the group consisting of C₄₋₁₈ alkyl.

In some embodiments, in R^{a} and R^{b}, the C₁₋₂₄ alkyl is each independently selected from the group consisting of straight C₁₋₂₄ alkyl and branched C₃₋₂₄ alkyl.

In some embodiments, the straight C₁₋₂₄ alkyl is selected from the group consisting of straight C₄₋₁₈ alkyl. In some embodiments, the straight C₄₋₁₈ alkyl is selected from the group consisting of C₇₋₁₃ alkyl.

In some embodiments, the branched C₃₋₂₄ alkyl is selected from the group consisting of branched C₄₋₁₈ alkyl. In some embodiments, the branched C₄₋₁₈ alkyl is selected from the group consisting of branched C₉₋₁₈ alkyl. In some embodiments, the branched C₃₋₂₄ alkyl is selected from the group consisting of 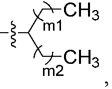 wherein, m1 and m2 are each independently an integer from 0 to 12, and the sum of m1 and m2 is no more than 21.

In some embodiments, R^{a} and R^{b} are each independently selected from the group consisting of : 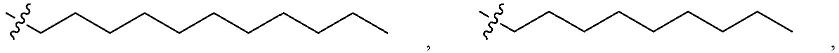 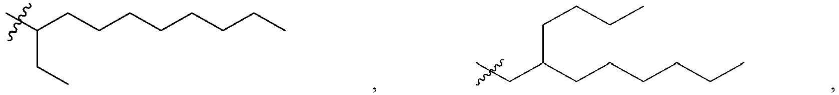 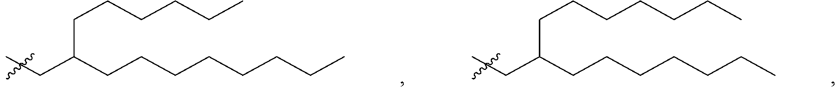 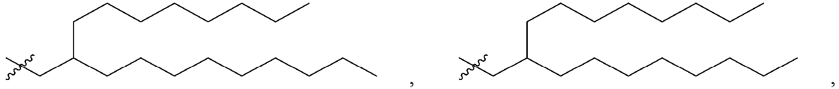 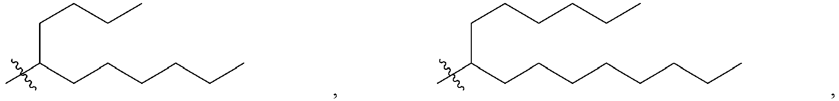 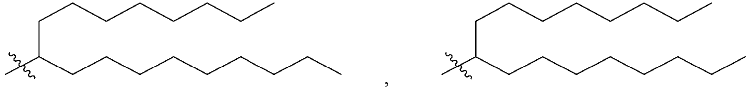 and

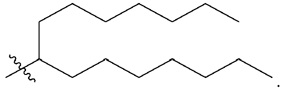

In some embodiments, R^{a} and R^{b} are each independently selected from the group consisting of branched C₄₋₁₈ alkenyl.

In some embodiments, R^{a} and R^{b} are each independently selected from the group consisting of straight C₄₋₁₈ alkenyl.

In some embodiments, R^{a} is selected from the group consisting of straight C₄₋₁₈ alkenyl, and R^{b} is selected from the group consisting of branched C₄₋₁₈ alkenyl.

In some embodiments, R^{a} is selected from the group consisting of branched C₄₋₁₈ alkenyl, and R^{b} is selected from the group consisting of branched C₄₋₁₈ alkenyl.

In some embodiments, in R^{a} and R^{b}, each C₂₋₂₄ alkenyl independently includes, but is not limited to C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, C₂₁ alkenyl, C₂₂ alkenyl, C₂₃ alkenyl, C₂₄ alkenyl. In some embodiments, in R^{a} and R^{b}, the C₂₋₂₄ alkenyl is selected from the group consisting of C₄₋₁₈ alkenyl.

In some embodiments, in R^{a} and R^{b}, the C₂₋₂₄ alkenyl is each independently selected from the group consisting of straight C₂₋₂₄ alkenyl and branched C₄₋₂₄ alkenyl.

In some embodiments, the straight C₂₋₂₄ alkenyl is selected from the group consisting of straight C₄₋₁₈ alkenyl.

In some embodiments, the branched C₄₋₂₄ alkenyl is selected from the group consisting of branched C₄₋₁₈ alkenyl.

In some embodiments, R^{c} is selected from the group consisting of NR^{c1}R^{c2}.

In some embodiments, R^{c} is NH₂.

In some embodiments, R^{c} is selected from the group consisting of NHR^{c1} and NHR^{c2}, wherein R^{c1} and R^{c2} are each independently selected from the group consisting of C₁₋₆ alkyl. In some embodiments, R^{c} is NHCH₃.

In some embodiments, in R^{c1} and R^{c2}, the C₁₋₆ alkyl is each independently selected from the group consisting of C₁₋₄ alkyl. In some embodiments, in R^{c1} and R^{c2}, the C₁₋₆ alkyl is each independently selected from the group consisting of methyl, ethyl, propyl, isopropyl, n-butyl, iso-butyl, sec-butyl and tert-butyl. In some embodiments, in R^{c1} and R^{c2}, the C₁₋₆ alkyl is methyl.

In some embodiments, R^{c} is selected from the group consisting of N⁺R^{c3}R^{c4}R^{c5}. In some embodiments, R^{c} is N⁺(CH₃)₃.

In some embodiments, R^{c} is selected from the group consisting of NH₂, NHCH₃ and N⁺(CH₃)₃.

In some embodiments, in R^{c3}, R^{c4} and R^{c5}, the C₁₋₆ alkyl is each independently selected from the group consisting of C₁₋₄ alkyl. In some embodiments, in R^{c3}, R^{c4} and R^{c5}, the C₁₋₆ alkyl is each independently selected from the group consisting of methyl, ethyl, propyl, isopropyl, n-butyl, iso-butyl, sec-butyl and tert-butyl. In some embodiments, in R^{c3}, R^{c4} and R^{c5}, the C₁₋₆ alkyl is methyl.

In some embodiments, the compound of formula I or a salt thereof is a compound of formula Ia or a salt thereof, 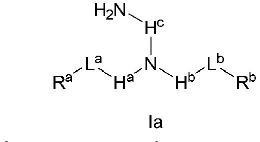 wherein, L^{a}, L^{b}, H^{a}, H^{b}, H^{c}, R^{a} and R^{b} are as defined in any embodiment of the compound of formula I.

In some embodiments, the compound of formula I or a salt thereof is selected from the group consisting of compounds of formulas IA, IB, IC, ID, IE and IF or salts thereof, 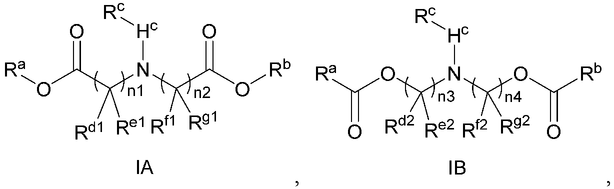 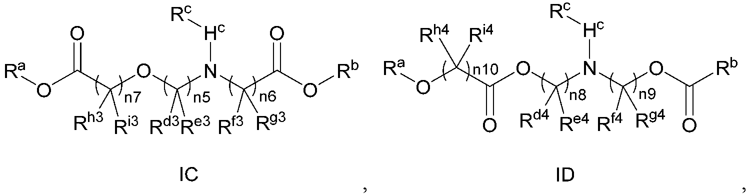 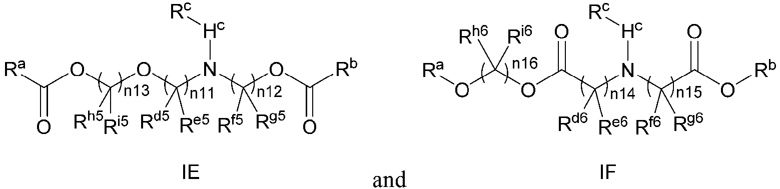
wherein, H^{c}, R^{a}, R^{b} and R^{c} are as defined in any embodiment of the compound of formula I;
R^{d1}, R^{d2}, R^{d3}, R^{d4}, R^{d5}, R^{d6}, R^{e1}, R^{e2}, R^{e3}, R^{e4}, R^{e5}, R^{e6}, R^{f1}, R^{f2}, R^{f3}, R^{f4}, R^{f5}, R^{f6}, R^{g1}, R^{g2}, R^{g3}, R^{g4}, R^{g5}, R^{g6}, R^{h3}, R^{h4}, R^{h5}, R^{h6}, Rⁱ³, Rⁱ⁴, Rⁱ⁵ and Rⁱ⁶ are each independently selected from the group consisting of hydrogen and C₁₋₆ alkyl;
n1, n2, n3, n4, n5, n6, n8, n9, n11, n12, n14 and n15 are each independently selected from the group consisting of 1, 2, 3, 4, 5, 6, 7, 8, 9 and 10;
n7, n10, n13 and n16 are each independently selected from the group consisting of 0, 1, 2, 3, 4, 5 and 6.

In some embodiments, the compound of formula I or a salt thereof is the above-described compound of formula IC or a salt thereof.

In some embodiments, R^{d1}, R^{d2}, R^{d3}, R^{d4}, R^{d5}, R^{d6}, R^{e1}, R^{e2}, R^{e3}, R^{e4}, R^{e5}, R^{e6}, R^{f1}, R^{f2}, R^{f3}, R^{f4}, R^{f5}, R^{f6}, R^{g1}, R^{g2}, R^{g3}, R^{g4}, R^{g5}, R^{g6}, R^{h3}, R^{h4}, R^{h5}, R^{h6}, Rⁱ³, Rⁱ⁴, Rⁱ⁵ and Rⁱ⁶ are hydrogen.

In some embodiments, n1, n2, n3, n4, n5, n6, n8, n9, n11, n12, n14 and n15 are each independently selected from the group consisting of 3, 4, 5, 6 and 7.

In some embodiments, n7, n10, n13 and n16 are each independently selected from the group consisting of 1, 2, 4, 5 and 6.

In some embodiments, n7, n10, n13 and n16 are each independently selected from the group consisting of 1 and 2.

In some embodiments, the compound of formula I is selected from the group consisting of the following structures,

| | |
|---|---|
| 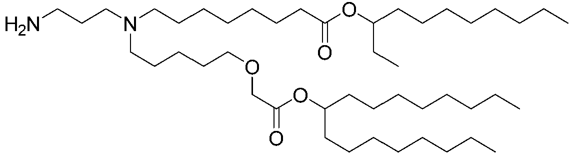 | I-1 |
| 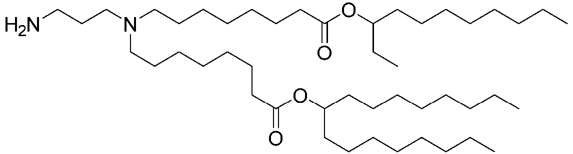 | I-2 |
| 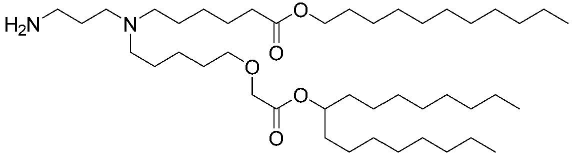 | I-3 |
| 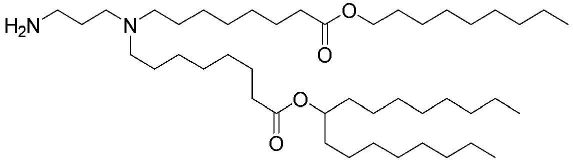 | I-4 |
| 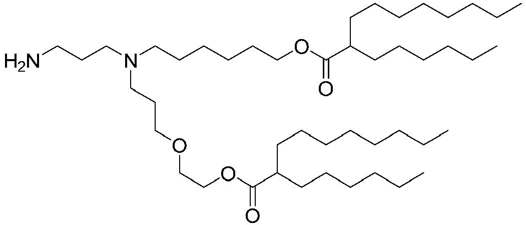 | I-5 |
| 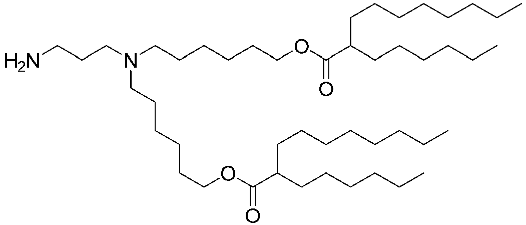 | I-6 |
| 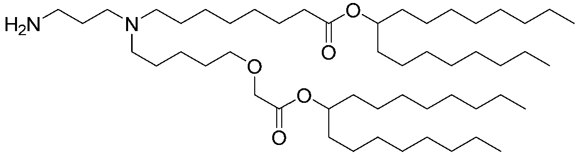 | I-7 |
| 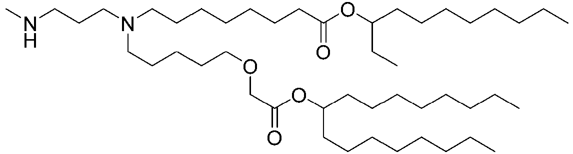 | I-8 |
| 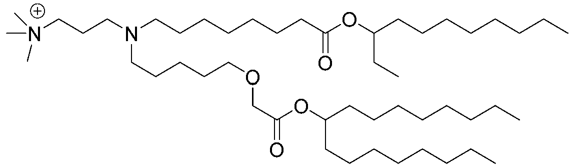 | I-9 |
| 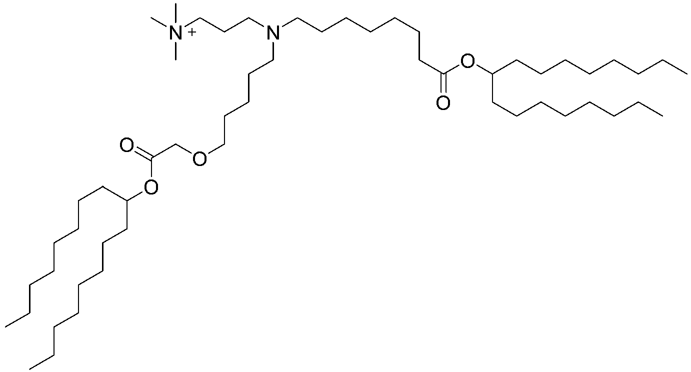 | I-10 |

In some embodiments, the lipid nanoparticle composition further comprises a compound of formula II or a salt thereof, 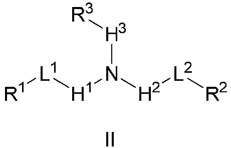
wherein, L¹ and L² are each independently selected from the group consisting of -O-, -C(O)-, -OC(O)-, -C(O)O-, -OC(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and at least one of L¹ and L² is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
H¹, H² and H³ are each independently selected from the group consisting of C₁₋₁₀ alkylene;
R¹ and R² are each independently selected from the group consisting of C₁₋₂₄ alkyl and C₂₋₂₄ alkenyl;
R³ is hydroxy.

In some embodiments, L¹ and L² are each independently selected from the group consisting of -OC(O)-, -C(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-.

In some embodiments, L¹ is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and L² is selected from the group consisting of -OC(O)- and -C(O)O-.

In some embodiments, L² is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and L¹ is selected from the group consisting of -OC(O)- and -C(O)O-.

In some embodiments, L¹ and L² are each independently selected from the group consisting of -OC(O)-, -C(O)O-, -OCH₂C(O)O-, -OC(O)CH₂O-, -OCH(CH₃)C(O)O-, -OC(O)CH(CH₃)O-, -OC(CH₃)₂C(O)O-, -OC(O)C(CH₃)₂O-, -OCH₂OC(O)-, -C(O)OCH₂O-, -O(CH₂)₂OC(O)-, -C(O)O(CH₂)₂O-, -O(CH₂)₄OC(O)- and -C(O)O(CH₂)₄O-.

In some embodiments, L¹ and L² are each independently selected from the group consisting of -OC(O)-, -C(O)O-, -OCH₂C(O)O-, -OC(O)CH₂O-, -OCH(CH₃)C(O)O-, -OC(O)CH(CH₃)O-, -OC(CH₃)₂C(O)O-, -OC(O)C(CH₃)₂O-, -OCH₂OC(O)-, -C(O)OCH₂O-, -O(CH₂)₂OC(O)-, -C(O)O(CH₂)₂O-, -O(CH₂)₄OC(O)- and -C(O)O(CH₂)₄O-, and at least one of L¹ and L² is selected from the group consisting of -OCH₂C(O)O-, -OC(O)CH₂O-, -OCH(CH₃)C(O)O-, -OC(O)CH(CH₃)O-, -OC(CH₃)₂C(O)O-, -OC(O)C(CH₃)₂O-, -OCH₂OC(O)-, -C(O)OCH₂O-, -O(CH₂)₂OC(O)-, -C(O)O(CH₂)₂O-, -O(CH₂)₄OC(O)- and -C(O)O(CH₂)₄O-.

In some embodiments, in L¹ and L², C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of straight or branched C₁₋₆ alkylene.

In some embodiments, in L¹ and L², C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of C₁ alkylene, C₂ alkylene, C₃ alkylene, C₄ alkylene, C₅ alkylene and C₆ alkylene.

In some embodiments, in L¹ and L², C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of C₁₋₄ alkylene.

In some embodiments, in L¹ and L², C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of methylene, ethylidene, n-propylidene, iso-propylidene, n-butylidene, sec-butylidene, iso-butylidene and tert-butylidene.

In some embodiments, in L¹ and L², C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of methylene and ethylidene.

In some embodiments, H¹ is selected from the group consisting of straight or branched C₁₋₁₀ alkylene. In some embodiments, H¹ is selected from the group consisting of straight or branched C₃₋₈ alkylene. In some embodiments, H¹ is selected from the group consisting of -(CH₂)₃-, -(CH₂)₄-, -(CH₂)₅-, -(CH₂)₆-, -(CH₂)₇- and -(CH₂)₈-.

In some embodiments, H² is selected from the group consisting of straight or branched C₁₋₁₀ alkylene. In some embodiments, H² is selected from the group consisting of straight or branched C₃₋₈ alkylene. In some embodiments, H² is selected from the group consisting of -(CH₂)₃-, -(CH₂)₄-, -(CH₂)₅-, -(CH₂)₆-, -(CH₂)₇- and -(CH₂)₈-.

In some embodiments, H³ is selected from the group consisting of straight or branched C₁₋₁₀ alkylene. In some embodiments, H³ is selected from the group consisting of straight or branched C₁₋₆ alkylene. In some embodiments, H³ is selected from the group consisting of straight or branched C₁₋₄ alkylene. In some embodiments, H³ is selected from the group consisting of -CH₂-, -(CH₂)₂-, -(CH₂)₃- and -(CH₂)₄-.

In some embodiments, in H¹, H² and H³, the C₁₋₁₀ alkylene is each independently straight C₁₋₁₀ alkylene.

In some embodiments, in H¹, H² and H³, the C₁₋₁₀ alkylene is each independently branched C₁₋₁₀ alkylene.

In some embodiments, in H¹, H² and H³, the C₁₋₁₀ alkylene is each independently selected from the group consisting of C₁ alkylene, C₂ alkylene, C₃ alkylene, C₄ alkylene, C₅ alkylene, C₆ alkylene, C₇ alkylene, C₈ alkylene, C₉ alkylene and C₁₀ alkylene.

In some embodiments, in H¹, H² and H³, the C₁₋₁₀ alkylene is each independently selected from the group consisting of C₃₋₈ alkylene.

In some embodiments, in H¹, H² and H³, the C₁₋₁₀ alkylene is each independently selected from the group consisting of C₁₋₆ alkylene. In some embodiments, in H¹, H² and H³, the C₁₋₁₀ alkylene is each independently selected from the group consisting of C₁₋₄ alkylene. In some embodiments, in H¹, H² and H³, the C₁₋₁₀ alkylene is each independently selected from the group consisting of methylene, ethylidene, n-propylidene, iso-propylidene, n-butylidene, sec-butylidene, iso-butylidene and tert-butylidene.

In some embodiments, R¹ and R² are each independently selected from the group consisting of straight or branched C₁₋₂₄ alkyl.

In some embodiments, R¹ and R² are each independently selected from the group consisting of straight or branched C₄₋₁₈ alkyl.

In some embodiments, R¹ is selected from the group consisting of straight C₄₋₁₈ alkyl, and R² is selected from the group consisting of branched C₄₋₁₈ alkyl.

In some embodiments, R¹ is selected from the group consisting of branched C₄₋₁₈ alkyl, and R² is selected from the group consisting of branched C₄₋₁₈ alkyl.

In some embodiments, R¹ is selected from the group consisting of straight C₄₋₁₈ alkyl, and R² is selected from the group consisting of straight C₄₋₁₈ alkyl.

In some embodiments, in R¹ and R², each C₁₋₂₄ alkyl independently includes, but is not limited to C₁ alkyl, C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, C₂₃ alkyl, C₂₄ alkyl. In some embodiments, in R¹ and R², the C₁₋₂₄ alkyl is each independently selected from the group consisting of C₄₋₁₈ alkyl.

In some embodiments, in R¹ and R², the C₁₋₂₄ alkyl is each independently selected from the group consisting of straight C₁₋₂₄ alkyl and branched C₃₋₂₄ alkyl.

In some embodiments, the straight C₁₋₂₄ alkyl is selected from the group consisting of straight C₄₋₁₈ alkyl. In some embodiments, the straight C₄₋₁₈ alkyl is selected from the group consisting of C₇₋₁₃ alkyl.

In some embodiments, the branched C₃₋₂₄ alkyl is selected from the group consisting of branched C₄₋₁₈ alkyl. In some embodiments, the branched C₄₋₁₈ alkyl is selected from the group consisting of branched C₉₋₁₈ alkyl. In some embodiments, the branched C₃₋₂₄ alkyl is selected from the group consisting of 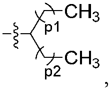 wherein, p1 and p2 are each independently an integer from 0 to 12, and the sum of p1 and p2 is no more than 21.

In some embodiments, R¹ and R² are each independently selected from the group consisting of: 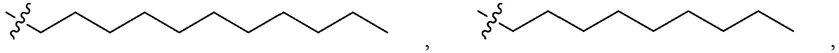 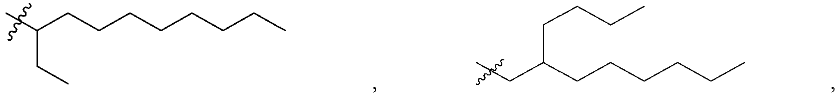 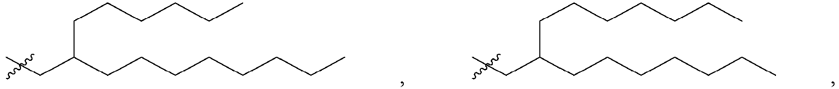 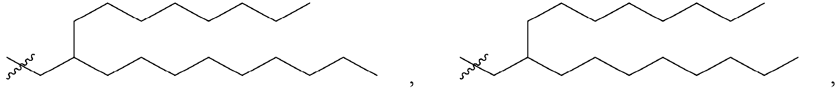 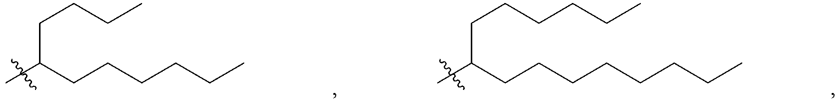 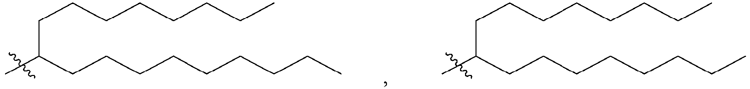 and

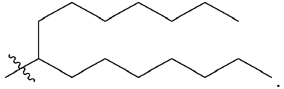

In some embodiments, R¹ and R² are each independently selected from the group consisting of branched C₄₋₁₈ alkenyl.

In some embodiments, R¹ and R² are each independently selected from the group consisting of straight C₄₋₁₈ alkenyl.

In some embodiments, R¹ is selected from the group consisting of straight C₄-₁₈ alkenyl, and R² is selected from the group consisting of branched C₄₋₁₈ alkenyl.

In some embodiments, R¹ is selected from the group consisting of branched C₄-₁₈ alkenyl, and R² is selected from the group consisting of branched C₄₋₁₈ alkenyl.

In some embodiments, in R¹ and R², the C₂₋₂₄ alkenyl includes, but is not limited to C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, C₂₁ alkenyl, C₂₂ alkenyl, C₂₃ alkenyl, C₂₄ alkenyl. In some embodiments, in R' and R², the C₂₋₂₄ alkenyl is selected from the group consisting of C₄₋₁₈ alkenyl.

In some embodiments, in R¹ and R², the C₂₋₂₄ alkenyl is selected from the group consisting of straight C₂₋₂₄ alkenyl and branched C₄₋₂₄ alkenyl.

In some embodiments, the straight C₂₋₂₄ alkenyl is selected from the group consisting of straight C₄₋₁₈ alkenyl.

In some embodiments, the branched C₄₋₂₄ alkenyl is selected from the group consisting of branched C₄₋₁₈ alkenyl.

In some embodiments, the compound of formula II or a salt thereof is selected from the group consisting of compounds of formulas IIA, IIB, IIC and IID or salts thereof, 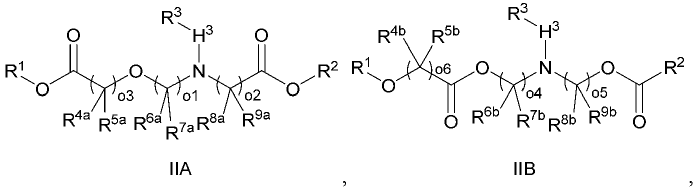 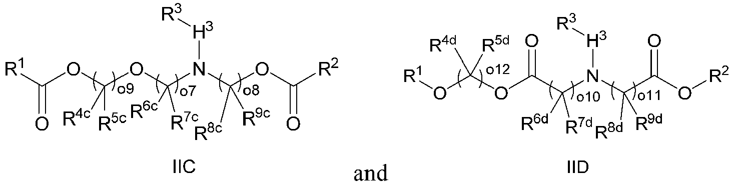
wherein, H³, R¹, R³ and R² are as defined in any embodiment of the compound of formula II;
R^{4a}, R^{4b}, R^{4c}, R^{4d}, R^{5a}, R^{5b}, R^{5c}, R^{5d}, R^{6a}, R^{6b}, R^{6c}, R^{6d}, R^{7a}, R^{7b}, R^{7c}, R^{7d}, R^{8a}, R^{8b}, R^{8c}, R^{8d}, R^{9a}, R^{9b}, R^{9c} and R^{9d} are each independently selected from the group consisting of hydrogen and C₁₋₆ alkyl;
o1, o2, o4, o5, o7, o8, o10 and o11 are each independently selected from the group consisting of 1, 2, 3, 4, 5, 6, 7, 8, 9 and 10;
o3, o6, o9 and o12 are each independently selected from the group consisting of 0, 1, 2, 3, 4, 5 and 6.

In some embodiments, the compound of formula II or a salt thereof is the above-described compound of formula IIA or a salt thereof.

In some embodiments, R^{4a}, R^{4b}, R^{4c}, R^{4d}, R^{5a}, R^{5b}, R^{5c}, R^{5d}, R^{6a}, R^{6b}, R^{6c}, R^{6d}, R^{7a}, R^{7b}, R^{7c}, R^{7d}, R^{8a}, R^{8b}, R^{8c}, R^{8d}, R^{9a}, R^{9b}, R^{9c} and R^{9d} are hydrogen.

In some embodiments, o1, o2, o4, o5, o7, o8, o10 and o11 are each independently selected from the group consisting of 2, 3, 4 and 5.

In some embodiments, o3, o6, o9 and o12 are each independently selected from the group consisting of 1, 2, 3 and 4.

In some embodiments, o3 is 1.

In some embodiments, o3 is 1, and R^{4a} and R^{5a} are each independently selected from hydrogen and methyl.

In some embodiments, the compound of formula II is Compound II-1,

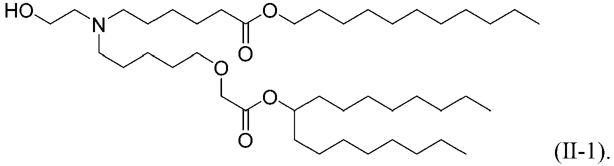

In some embodiments, the molar percentage of the compound of formula I or a salt thereof to the lipid nanoparticle composition ranges from 5% to 80% (for example 5%, 10%, 11.25%, 12%, 15%, 17.5%, 20%, 24%, 25%, 30%, 35%, 36%, 40%, 45%, 50%, 55%, 60%, 65%, 70%, 75%, 80% and any value among them). In some embodiments, the molar percentage of the compound of formula I or a salt thereof to the lipid nanoparticle composition ranges from 10% to 80%. In some embodiments, the molar percentage of the compound of formula I or a salt thereof to the lipid nanoparticle composition ranges from 10% to 60% (for example 11.25%, 12%, 17.5%, 24%, 25%, 36% or 50%). In some embodiments, the molar percentage of the compound of formula I or a salt thereof to the lipid nanoparticle composition ranges from 10% to 40%. In some embodiments, the molar percentage of the compound of formula I or a salt thereof to the lipid nanoparticle composition ranges from 10% to 30%. In some embodiments, the molar percentage of the compound of formula I or a salt thereof to the lipid nanoparticle composition ranges from 15% to 60%. In some embodiments, the molar percentage of the compound of formula I or a salt thereof to the lipid nanoparticle composition ranges from 15% to 35%. In some embodiments, the molar percentage of the compound of formula I or a salt thereof to the lipid nanoparticle composition ranges from 40% to 60%.

In some embodiments, the molar percentage of the compound of formula II or a salt thereof to the lipid nanoparticle composition ranges from 5% to 50% (for example 5%, 10%, 12%, 15%, 20%, 24%, 25%, 30%, 35%, 36%, 37.5%, 40%, 43.75%, 45%, 50% and any value among them). In some embodiments, the molar percentage of the compound of formula II or a salt thereof to the lipid nanoparticle composition ranges from 15% to 35%. In some embodiments, the molar percentage of the compound of formula II or a salt thereof to the lipid nanoparticle composition ranges from 10% to 45% (for example 12%, 24%, 25%, 30%, 36%, 37.5% or 43.75%). In some embodiments, the molar percentage of the compound of formula II or a salt thereof to the lipid nanoparticle composition ranges from 20% to 45%.

In some embodiments, the lipid nanoparticle composition comprises a phospholipid.

In some embodiments, the phospholipid is selected from the group consisting of 1,2-dipalmitoyl-sn-glycero-3-o-4'-(N,N,N-trimethyl)homoserine (DGTS), 1,2-dioleoyl-sn-glycero-3-phosphoethanolamine (DOPE), and 1,2-distearoyl-sn-glycero-3-phosphocholine (DSPC). In some embodiments, the phospholipid is1,2-distearoyl-sn-glycero-3-phosphocholine (DSPC).

In some embodiments, the molar percentage of the phospholipid to the lipid nanoparticle composition ranges from 1% to 25% (for example 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25% and any value among them). In some embodiments, the molar percentage of the phospholipid to the lipid nanoparticle composition ranges from 1% to 15% (for example 5% or 10%). In some embodiments, the molar percentage of the phospholipid to the lipid nanoparticle composition ranges from 1% to 8%. In some embodiments, the molar percentage of the phospholipid to the lipid nanoparticle composition ranges from 8% to 15%.

In some embodiments, the lipid nanoparticle composition further comprises a steroid.

In some embodiments, the steroid is cholesterol.

In some embodiments, the molar percentage of the steroid to the lipid nanoparticle composition ranges from 10% to 50% (for example 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, 25%, 25.5%, 26%, 26.5%, 27%, 27.55%, 28%, 28.5%, 29%, 29.5%, 30%, 30.5%, 31%, 31.5%, 32%, 32.5%, 33%, 33.5%, 34%, 34.5%, 35%, 35.5%, 36%, 36.5%, 37%, 37.5%, 38%, 38.5%, 39%, 39.5%, 40%, 40.5%, 41%, 41.5%, 42%, 42.5%, 43%, 43.5%, 44%, 44.5%, 45%, 45.5%, 46%, 46.5%, 47%, 47.55%, 48%, 48.5%, 49%, 49.5%, 50% and any value among them). In some embodiments, the molar percentage of the steroid to the lipid nanoparticle composition ranges from 10% to 45% (for example 18.5%, 38.5% or 40.5%). In some embodiments, the molar percentage of the steroid to the lipid nanoparticle composition ranges from 10% to 25%. In some embodiments, the molar percentage of the steroid to the lipid nanoparticle composition ranges from 30% to 45%.

In some embodiments, the lipid nanoparticle composition further comprises a pegylated lipid.

In some embodiments, the pegylated lipid comprises a PEG moiety of about 1000 to about 10,000 Daltons (for example 1000 Daltons, 2000 Daltons, 3000 Daltons, 4000 Daltons, 5000 Daltons, 6000 Daltons, 7000 Daltons, 8000 Daltons, 9000 Daltons or 10000 Daltons).

In some embodiments, the pegylated lipid is a pegylated diacylglycerol. In some embodiments, the pegylated lipid is 1,2-dimyristoyl-rac-glycero-3-methoxypolyethylene glycol (DMG-PEG). In some embodiments, the pegylated lipid is DMG-PEG 2000.

In some embodiments, the molar percentage of the pegylated lipid to the lipid nanoparticle composition ranges from 0.5% to 10% (for example 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%). In some embodiments, the molar percentage of the pegylated lipid to the lipid nanoparticle composition ranges from 0.5% to 5% (for example 1.5%).

In some embodiments, the lipid nanoparticle composition comprises the above-described compound of formula I or a salt thereof, a phospholipid, a steroid, and a pegylated lipid.

In some embodiments, the lipid nanoparticle composition comprises the above-described compound of formula I or a salt thereof, a phospholipid, a steroid, and a pegylated lipid, wherein the molar percentage of the compound of formula I or a salt thereof to the lipid nanoparticle composition ranges from 10% to 80%; the phospholipid is selected from the group consisting of DGTS, DOPE and DSPC, and the molar percentage of the phospholipid to the lipid nanoparticle composition ranges from 1% to 25%; the steroid is cholesterol, and the molar percentage of the steroid to the lipid nanoparticle composition ranges from 10% to 50%; the pegylated lipid is a pegylated diacylglycerol, and the molar percentage of the pegylated lipid to the lipid nanoparticle composition ranges from 0.5% to 10%.

In some embodiments, the lipid nanoparticle composition comprises the above-described compound of formula Ia or a salt thereof, a phospholipid, a steroid, and a pegylated lipid, wherein the molar percentage of the compound of formula Ia or a salt thereof to the lipid nanoparticle composition ranges from 10% to 80%; the phospholipid is selected from the group consisting of DGTS, DOPE and DSPC, and the molar percentage of the phospholipid to the lipid nanoparticle composition ranges from 1% to 25%; the steroid is cholesterol, and the molar percentage of the steroid to the lipid nanoparticle composition ranges from 10% to 50%; the pegylated lipid is a pegylated diacylglycerol, and the molar percentage of the pegylated lipid to the lipid nanoparticle composition ranges from 0.5% to 10%.

In some embodiments, the lipid nanoparticle composition comprises the above-described compound of formula Ia or a salt thereof, DSPC, cholesterol, and DMG-PEG, wherein the molar percentage of the compound of formula Ia or a salt thereof to the lipid nanoparticle composition ranges from 15% to 60%; the molar percentage of the DSPC to the lipid nanoparticle composition ranges from 1% to 15%; the molar percentage of the cholesterol to the lipid nanoparticle composition ranges from 30% to 45%; and the molar percentage of the DMG-PEG to the lipid nanoparticle composition ranges from 0.5% to 5%.

In some embodiments, the lipid nanoparticle composition comprises any one of the above-described Compounds I-1 to I-10 or a salt thereof, DSPC, cholesterol, and DMG-PEG, wherein the molar percentage of any one of the Compounds I-1 to I-10 or a salt thereof to the lipid nanoparticle composition ranges from 15% to 60%; the molar percentage of the DSPC to the lipid nanoparticle composition ranges from 1% to 15%; the molar percentage of the cholesterol to the lipid nanoparticle composition ranges from 30% to 45%; and the molar percentage of the DMG-PEG to the lipid nanoparticle composition ranges from 0.5% to 5%.

In some embodiments, the lipid nanoparticle composition comprises the above-described Compound I-1 or a salt thereof, DSPC, cholesterol, and DMG-PEG, wherein the molar ratio of the Compound I-1 or the salt thereof, DSPC, cholesterol and DMG-PEG is 50:10:38.5:1.5.

In some embodiments, the lipid nanoparticle composition comprises the above-described compound of formula I or a salt thereof, the above-described compound of formula II or a salt thereof, a phospholipid, a steroid, and a pegylated lipid.

In some embodiments, the lipid nanoparticle composition comprises the above-described compound of formula I or a salt thereof, the above-described compound of formula II or a salt thereof, a phospholipid, a steroid, and a pegylated lipid, wherein the molar percentage of the compound of formula I or a salt thereof to the lipid nanoparticle composition ranges from 10% to 80%; the molar percentage of the compound of formula II or a salt thereof to the lipid nanoparticle composition ranges from 5% to 50%; the phospholipid is selected from the group consisting of DGTS, DOPE and DSPC, and the molar percentage of the phospholipid to the lipid nanoparticle composition ranges from 1% to 25%; the steroid is cholesterol, and the molar percentage of the steroid to the lipid nanoparticle composition ranges from 10% to 50%; the pegylated lipid is a pegylated diacylglycerol, and the molar percentage of the pegylated lipid to the lipid nanoparticle composition ranges from 0.5% to 10%.

In some embodiments, the lipid nanoparticle composition comprises the above-described compound of formula I or a salt thereof, the above-described compound of formula II or a salt thereof, DSPC, cholesterol, and DMG-PEG, wherein the molar percentage of the compound of formula I or a salt thereof to the lipid nanoparticle composition ranges from 15% to 60%; the molar percentage of the compound of formula II or a salt thereof to the lipid nanoparticle composition ranges from 15% to 35%; the molar percentage of the DSPC to the lipid nanoparticle composition ranges from 1% to 15%; the molar percentage of the cholesterol to the lipid nanoparticle composition ranges from 10% to 25% or from 30% to 45%; and the molar percentage of the DMG-PEG to the lipid nanoparticle composition ranges from 0.5% to 5%.

In some embodiments, the lipid nanoparticle composition comprises the above-described compound of formula Ia or a salt thereof, the above-described compound of formula II or a salt thereof (for example Compound II-1 or a salt thereof), DSPC, cholesterol, and DMG-PEG, wherein the molar percentage of the compound of formula I or a salt thereof to the lipid nanoparticle composition ranges from 10% to 60%; the molar percentage of the compound of formula II or a salt thereof to the lipid nanoparticle composition ranges from 10% to 45%; the molar percentage of the DSPC to the lipid nanoparticle composition ranges from 1% to 15%; the molar percentage of the cholesterol to the lipid nanoparticle composition ranges from 10% to 45%; and the molar percentage of the DMG-PEG to the lipid nanoparticle composition ranges from 0.5% to 5%.

In some embodiments, the lipid nanoparticle composition comprises the above-described compound of formula Ia or a salt thereof, the above-described compound of formula II or a salt thereof, DSPC, cholesterol, and DMG-PEG, wherein the molar percentage of the compound of formula I or a salt thereof to the lipid nanoparticle composition ranges from 15% to 60%; the molar percentage of the compound of formula II or a salt thereof to the lipid nanoparticle composition ranges from 15% to 35%; the molar percentage of the DSPC to the lipid nanoparticle composition ranges from 1% to 15%; the molar percentage of the cholesterol to the lipid nanoparticle composition ranges from 10% to 25% or from 30% to 45%; and the molar percentage of the DMG-PEG to the lipid nanoparticle composition ranges from 0.5% to 5%.

In some embodiments, the lipid nanoparticle composition comprises the above-described compound of formula Ia or a salt thereof, the above-described Compound II-1 or a salt thereof, DSPC, cholesterol, and DMG-PEG, wherein the molar percentage of the compound of formula Ia or a salt thereof to the lipid nanoparticle composition ranges from 15% to 60%; the molar percentage of the Compound II-1 or a salt thereof to the lipid nanoparticle composition ranges from 15% to 35%; the molar percentage of the DSPC to the lipid nanoparticle composition ranges from 1% to 15%; the molar percentage of the cholesterol to the lipid nanoparticle composition ranges from 10% to 25% or from 30% to 45%; and the molar percentage of the DMG-PEG to the lipid nanoparticle composition ranges from 0.5% to 5%.

In some embodiments, the lipid nanoparticle composition comprises any one of the above-described Compounds I-1 to I-10 or a salt thereof, the above-described Compound II-1 or a salt thereof, DSPC, cholesterol, and DMG-PEG, wherein the molar percentage of any one of the Compounds I-1 to I-10 or a salt thereof to the lipid nanoparticle composition ranges from 10% to 60%; the molar percentage of the Compound II-1 or a salt thereof to the lipid nanoparticle composition ranges from 10% to 45%; the molar percentage of the DSPC to the lipid nanoparticle composition ranges from 1% to 15%; the molar percentage of the cholesterol to the lipid nanoparticle composition ranges from 10% to 45%; and the molar percentage of the DMG-PEG to the lipid nanoparticle composition ranges from 0.5% to 5%.

In some embodiments, the lipid nanoparticle composition comprises any one of the above-described Compounds I-1 to I-10 or a salt thereof, the above-described Compound II-1 or a salt thereof, DSPC, cholesterol, and DMG-PEG, wherein the molar percentage of any one of the Compounds I-1 to I-10 or a salt thereof to the lipid nanoparticle composition ranges from 15% to 60%; the molar percentage of the Compound II-1 or a salt thereof to the lipid nanoparticle composition ranges from 15% to 35%; the molar percentage of the DSPC to the lipid nanoparticle composition ranges from 1% to 15%; the molar percentage of the cholesterol to the lipid nanoparticle composition ranges from 10% to 25% or from 30% to 45%; and the molar percentage of the DMG-PEG to the lipid nanoparticle composition ranges from 0.5% to 5%.

In some embodiments, the lipid nanoparticle composition comprises the above-described Compound I-1 or a salt thereof, the above-described Compound II-1 or a salt thereof, DSPC, cholesterol, and DMG-PEG, wherein the molar ratio of the Compound I-1 or the salt thereof, Compound II-1, DSPC, cholesterol and DMG-PEG is 25:25:10:38.5:1.5, or 50:25:5:18.5:1.5.

In some embodiments, the lipid nanoparticle composition comprises the above-described Compound I-1 or a salt thereof, the above-described Compound II-1 or a salt thereof, DSPC, cholesterol, and DMG-PEG, wherein the molar ratio of the Compound I-1 or the salt thereof, Compound II-1, DSPC, cholesterol and DMG-PEG is 11.25:43.75:5:38.5:1.5, 12:36:10:40.5:1.5, 17.5:37.5:5:38.5:1.5, 24:24:10:40.5:1.5, 25:30:5:38.5:1.5, or 36:12:10:40.5:1.5.

In some embodiments, the lipid nanoparticle composition comprises the above-described Compound I-2 or a salt thereof, the above-described Compound II-1 or a salt thereof, DSPC, cholesterol, and DMG-PEG, wherein the molar ratio of the Compound I-2 or the salt thereof, Compound II-1, DSPC, cholesterol and DMG-PEG is 25:25:10:38.5:1.5, or 50:25:5:18.5:1.5.

In some embodiments, the lipid nanoparticle composition comprises the above-described Compound I-8 or a salt thereof, the above-described Compound II-1 or a salt thereof, DSPC, cholesterol, and DMG-PEG, wherein the molar ratio of the Compound I-8 or the salt thereof, Compound II-1, DSPC, cholesterol and DMG-PEG is 50:25:5:18.5:1.5.

In some embodiments, the lipid nanoparticle composition comprises the above-described Compound I-10 or a salt thereof, the above-described Compound II-1 or a salt thereof, DSPC, cholesterol, and DMG-PEG, wherein the molar ratio of the Compound I-10 or the salt thereof, Compound II-1, DSPC, cholesterol and DMG-PEG is 50:25:5:18.5:1.5.

The present disclosure provides a lipid nanoparticle composition comprising a primary amine lipid.

In some embodiments, the primary amine lipid is the compound of formula Ia or a salt thereof described in any one of the above embodiments, 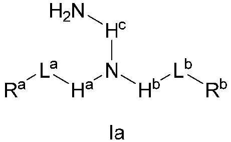 wherein, L^{a}, L^{b}, H^{a}, H^{b}, H^{c}, R^{a} and R^{b} are as defined in any embodiment of the aforementioned compound of formula I.

In some embodiments, the compound of formula Ia is any one selected from the group consisting of the following compounds,

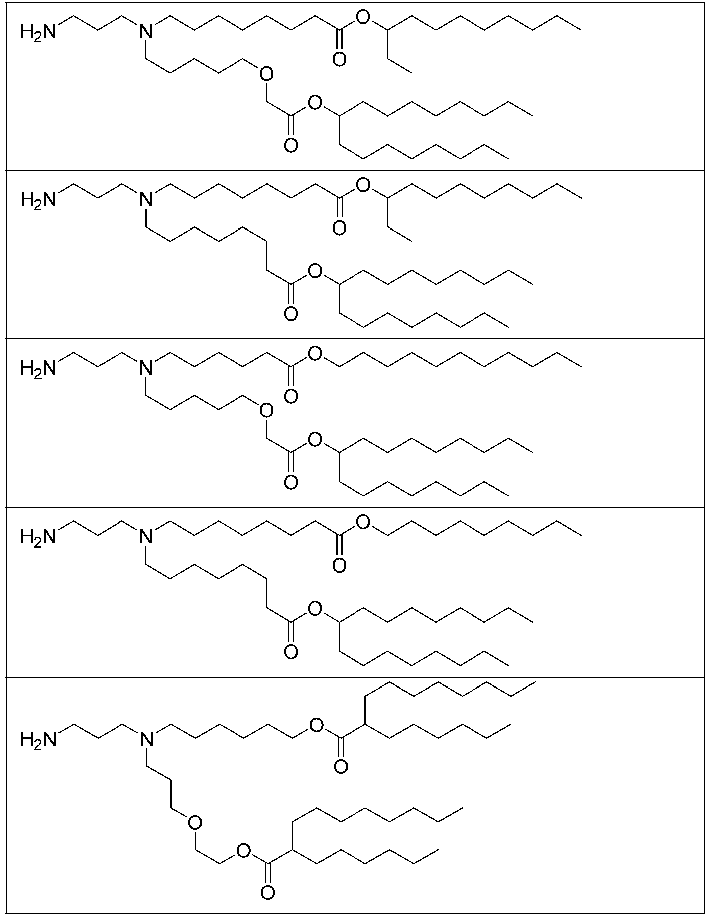

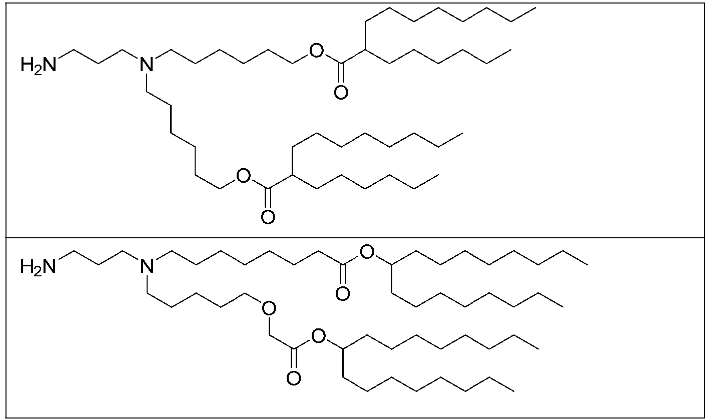

In some embodiments, the lipid nanoparticle composition further comprises the compound of formula II or a salt thereof described in any one of the above embodiments, 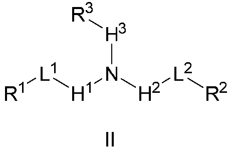 wherein, L¹, L², H¹, H², H³, R¹, R² and R³ are as defined in any one of the aforementioned embodiments.

In some embodiments, the compound of formula II is Compound II-1,

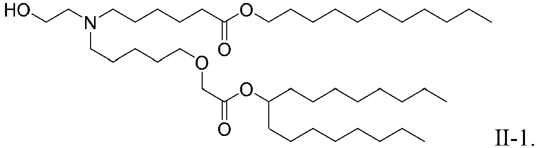

In some embodiments, the molar percentage of the primary amine lipid to the lipid nanoparticle composition ranges from 5% to 80% (for example 5%, 10%, 11.25%, 12%, 15%, 17.5%, 20%, 24%, 25%, 30%, 35%, 36%, 40%, 45%, 50%, 55%, 60%, 65%, 70%, 75%, 80% and any value among them). In some embodiments, the molar percentage of the primary amine lipid to the lipid nanoparticle composition ranges from 10% to 80%. In some embodiments, the molar percentage of the primary amine lipid to the lipid nanoparticle composition ranges from 10% to 60% (for example 11.25%, 12%, 17.5%, 24%, 25%, 36% or 50%). In some embodiments, the molar percentage of the primary amine lipid to the lipid nanoparticle composition ranges from 10% to 40%. In some embodiments, the molar percentage of the primary amine lipid to the lipid nanoparticle composition ranges from 10% to 30%. In some embodiments, the molar percentage of the primary amine lipid to the lipid nanoparticle composition ranges from 15% to 60%. In some embodiments, the molar percentage of the primary amine lipid to the lipid nanoparticle composition ranges from 15% to 35%. In some embodiments, the molar percentage of the primary amine lipid to the lipid nanoparticle composition ranges from 40% to 60%.

In some embodiments, the molar percentage of the compound of formula II or a salt thereof to the lipid nanoparticle composition ranges from 5% to 50% (for example 5%, 10%, 12%, 15%, 20%, 24%, 25%, 30%, 35%, 36%, 37.5%, 40%, 43.75%, 45%, 50% and any value among them). In some embodiments, the molar percentage of the compound of formula II or a salt thereof to the lipid nanoparticle composition ranges from 15% to 35%. In some embodiments, the molar percentage of the compound of formula II or a salt thereof to the lipid nanoparticle composition ranges from 10% to 45% (for example 12%, 24%, 25%, 30%, 36%, 37.5% or 43.75%). In some embodiments, the molar percentage of the compound of formula II or a salt thereof to the lipid nanoparticle composition ranges from 20% to 45%.

In some embodiments, the lipid nanoparticle composition comprises a phospholipid.

In some embodiments, the phospholipid is selected from the group consisting of 1,2-dipalmitoyl-sn-glycero-3-o-4'-(N,N,N-trimethyl)homoserine (DGTS), 1,2-dioleoyl-sn-glycero-3-phosphoethanolamine (DOPE), and 1,2-distearoyl-sn-glycero-3-phosphocholine (DSPC). In some embodiments, the phospholipid is1,2-distearoyl-sn-glycero-3-phosphocholine (DSPC).

In some embodiments, the molar percentage of the phospholipid to the lipid nanoparticle composition ranges from 1% to 25% (for example 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25% and any value among them). In some embodiments, the molar percentage of the phospholipid to the lipid nanoparticle composition ranges from 1% to 15% (for example 5% or 10%). In some embodiments, the molar percentage of the phospholipid to the lipid nanoparticle composition ranges from 1% to 8%. In some embodiments, the molar percentage of the phospholipid to the lipid nanoparticle composition ranges from 8% to 15%.

In some embodiments, the lipid nanoparticle composition further comprises a steroid.

In some embodiments, the steroid is cholesterol.

In some embodiments, the molar percentage of the steroid to the lipid nanoparticle composition ranges from 10% to 50% (for example 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 20.5%, 21%, 21.5%, 22%, 22.5%, 23%, 23.5%, 24%, 24.5%, 25%, 25.5%, 26%, 26.5%, 27%, 27.55%, 28%, 28.5%, 29%, 29.5%, 30%, 30.5%, 31%, 31.5%, 32%, 32.5%, 33%, 33.5%, 34%, 34.5%, 35%, 35.5%, 36%, 36.5%, 37%, 37.55%, 38%, 38.5%, 39%, 39.5%, 40%, 40.5%, 41%, 41.5%, 42%, 42.5%, 43%, 43.5%, 44%, 44.5%, 45%, 45.5%, 46%, 46.5%, 47%, 47.55%, 48%, 48.5%, 49%, 49.5%, 50% and any value among them). In some embodiments, the molar percentage of the steroid to the lipid nanoparticle composition ranges from 10% to 45% (for example 18.5%, 38.5% or 40.5%). In some embodiments, the molar percentage of the steroid to the lipid nanoparticle composition ranges from 10% to 25%. In some embodiments, the molar percentage of the steroid to the lipid nanoparticle composition ranges from 30% to 45%.

In some embodiments, the lipid nanoparticle composition further comprises a pegylated lipid.

In some embodiments, the pegylated lipid comprises a PEG moiety of about 1000 to about 10,000 Daltons (for example 1000 Daltons, 2000 Daltons, 3000 Daltons, 4000 Daltons, 5000 Daltons, 6000 Daltons, 7000 Daltons, 8000 Daltons, 9000 Daltons or 10000 Daltons).

In some embodiments, the pegylated lipid is a pegylated diacylglycerol. In some embodiments, the pegylated lipid is 1,2-dimyristoyl-rac-glycero-3-methoxypolyethylene glycol (DMG-PEG). In some embodiments, the pegylated lipid is DMG-PEG 2000.

In some embodiments, the molar percentage of the pegylated lipid to the lipid nanoparticle composition ranges from 0.5% to 10% (for example 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%). In some embodiments, the molar percentage of the pegylated lipid to the lipid nanoparticle composition ranges from 0.5% to 5% (for example 1.5%).

In some embodiments, the lipid nanoparticle composition comprises the above-described primary amine lipid, a phospholipid, a steroid, and a pegylated lipid, wherein the molar percentage of the primary amine lipid to the lipid nanoparticle composition ranges from 10% to 80%; the phospholipid is selected from the group consisting of DGTS, DOPE and DSPC, and the molar percentage of the phospholipid to the lipid nanoparticle composition ranges from 1% to 25%; the steroid is cholesterol, and the molar percentage of the steroid to the lipid nanoparticle composition ranges from 10% to 50%; the pegylated lipid is a pegylated diacylglycerol, and the molar percentage of the pegylated lipid to the lipid nanoparticle composition ranges from 0.5% to 10%.

In some embodiments, the lipid nanoparticle composition comprises the above-described primary amine lipid, DSPC, cholesterol, and DMG-PEG, wherein the molar percentage of the primary amine lipid to the lipid nanoparticle composition ranges from 15% to 60%; the molar percentage of the DSPC to the lipid nanoparticle composition ranges from 1% to 15%; the molar percentage of the cholesterol to the lipid nanoparticle composition ranges from 30% to 45%; and the molar percentage of the DMG-PEG to the lipid nanoparticle composition ranges from 0.5% to 5%.

In some embodiments, the lipid nanoparticle composition comprises the above-described primary amine lipid, the above-described compound of formula II, a phospholipid, a steroid, and a pegylated lipid, wherein the molar percentage of the primary amine lipid to the lipid nanoparticle composition ranges from 10% to 80%; the molar percentage of the compound of formula II or a salt thereof to the lipid nanoparticle composition ranges from 5% to 50%; the phospholipid is selected from the group consisting of DGTS, DOPE and DSPC, and the molar percentage of the phospholipid to the lipid nanoparticle composition ranges from 1% to 25%; the steroid is cholesterol, and the molar percentage of the steroid to the lipid nanoparticle composition ranges from 10% to 50%; the pegylated lipid is a pegylated diacylglycerol, and the molar percentage of the pegylated lipid to the lipid nanoparticle composition ranges from 0.5% to 10%.

In some embodiments, the lipid nanoparticle composition comprises the above-described primary amine lipid, the above-described compound of formula II, DSPC, cholesterol, and DMG-PEG, wherein the molar percentage of the primary amine lipid to the lipid nanoparticle composition ranges from 15% to 60%; the molar percentage of the compound of formula II or a salt thereof to the lipid nanoparticle composition ranges from 15% to 35%; the molar percentage of the DSPC to the lipid nanoparticle composition ranges from 1% to 15%; the molar percentage of the cholesterol to the lipid nanoparticle composition ranges from 10% to 25% or from 30% to 45%; and the molar percentage of the DMG-PEG to the lipid nanoparticle composition ranges from 0.5% to 5%.

In some embodiments, the lipid nanoparticle composition comprises the above-described primary amine lipid, the above-described compound of formula II, DSPC, cholesterol, and DMG-PEG, wherein the molar percentage of the primary amine lipid to the lipid nanoparticle composition ranges from 10% to 60%; the molar percentage of the compound of formula II or a salt thereof to the lipid nanoparticle composition ranges from 10% to 45%; the molar percentage of the DSPC to the lipid nanoparticle composition ranges from 1% to 15%; the molar percentage of the cholesterol to the lipid nanoparticle composition ranges from 10% to 45%; and the molar percentage of the DMG-PEG to the lipid nanoparticle composition ranges from 0.5% to 5%.

In some embodiments, the lipid nanoparticle composition of the present disclosure further comprises a therapeutic agent selected from the group consisting of a small molecule, a protein, and a nucleic acid.

In some embodiments, the therapeutic agent is a nucleic acid.

In some embodiments, the nucleic acid is a theraputic nucleic acid.

In some embodiments, the nucleic acid is selected from the group consisting of a siRNA, miRNA, a pri-□miRNA, a messenger RNA (mRNA), a clustered regularly interspaced short palindromic repeats (CRISPR)-associated nucleic acid, a single guide RNA (sgRNA), a CRISPR-□RNA (crRNA), a trans-activating crRNA (tracrRNA), a plasmid DNA (pDNA), a transfer RNA (tRNA), an antisense oligonucleotide (ASO), a guide RNA (gRNA), a double-stranded DNA (dsDNA), a single-stranded DNA (ssDNA), a single-stranded RNA (ssRNA), and a double-stranded RNA (dsRNA). In some embodiments, the nucleic acid is an mRNA.

In some embodiments, the weight ratio of the lipid nanoparticle composition to the nucleic acid ranges from about 1:1 to about 100:1.

In some embodiments, the composition of the present disclosure preferentially delivers a therapeutic agent to a target organ selected from the group consisting of lung, heart, liver, spleen, and stomach.

In some embodiments, the target organ is lung.

In some embodiments, the distribution level of the therapeutic agent in lung is higher than that in other organ.

The present disclosure provides a compound of formula III or a salt thereof, 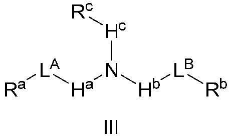 wherein,
L^{A} and L^{B} are each independently selected from the group consisting of -O-, -C(O)-, -OC(O)-, -C(O)O-, -OC(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and at least one of L^{A} and L^{B} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, wherein C₁₋₆ alkylene is not n-propylidene;
H^{a}, H^{b}, H^{c}, R^{a}, R^{b} and R^{c} are as defined in any embodiment of the compound of formula I.

In some embodiments, L^{A} and L^{B} are each independently selected from the group consisting of -OC(O)-, -C(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, wherein C₁₋₆ alkylene is not n-propylidene.

In some embodiments, at least one (for example one) of L^{A} and L^{B} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-.

In some embodiments, L^{A} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and L^{B} is selected from the group consisting of -OC(O)- and -C(O)O-, wherein C₁₋₆ alkylene is not n-propylidene.

In some embodiments, L^{B} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and L^{A} is selected from the group consisting of -OC(O)- and -C(O)O-, wherein C₁₋₆ alkylene is not n-propylidene.

In some embodiments, L^{A} and L^{B} are each independently selected from the group consisting of -OC(O)-, -C(O)O-, -OCH₂C(O)O-, -OC(O)CH₂O-, -OCH₂OC(O)-, -C(O)OCH₂O-, -O(CH₂)₂OC(O)- and -C(O)O(CH₂)₂O-.

In some embodiments, L^{A} and L^{B} are each independently selected from the group consisting of -OC(O)-, -C(O)O-, -OCH₂C(O)O-, -OC(O)CH₂O-, -OCH₂OC(O)-, -C(O)OCH₂O-, -O(CH₂)₂OC(O)- and -C(O)O(CH₂)₂O-, and at least one (for example one) of L^{A} and L^{B} is selected from the group consisting of -OCH₂C(O)O-, -OC(O)CH₂O-, -OCH₂OC(O)-, -C(O)OCH₂O-, -O(CH₂)₂OC(O)- and or -C(O)O(CH₂)₂O-.

In some embodiments, in L^{A} and L^{B}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of straight or branched C₁₋₆ alkylene.

In some embodiments, in L^{A} and L^{B}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of C₁ alkylene, C₂ alkylene, C₃ alkylene, C₄ alkylene, C₅ alkylene and C₆ alkylene.

In some embodiments, in L^{A} and L^{B}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of C₁₋₄ alkylene.

In some embodiments, in L^{A} and L^{B}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of methylene, ethylidene, iso-propylidene, n-butylidene, sec-butylidene, iso-butylidene and tert-butylidene.

In some embodiments, in L^{A} and L^{B}, C₁₋₆ alkylene of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O- is independently selected from the group consisting of methylene and ethylidene.

In some embodiments, the compound of formula III or a salt thereof is a compound of formula IIIa or a salt thereof, 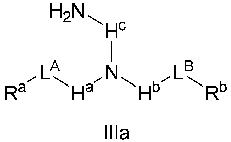 wherein, L^{A}, L^{B}, H^{a}, H^{b}, H^{c}, R^{a} and R^{b} are as defined in any embodiment of the compound of formula III.

In some embodiments, the compound of formula III or a salt thereof is selected from the group consisting of compounds of formulas IIIA, IIIB, IIIC and IIID or a salt thereof, 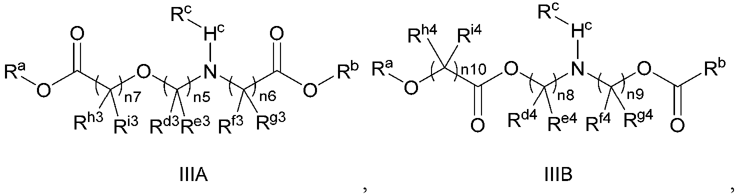 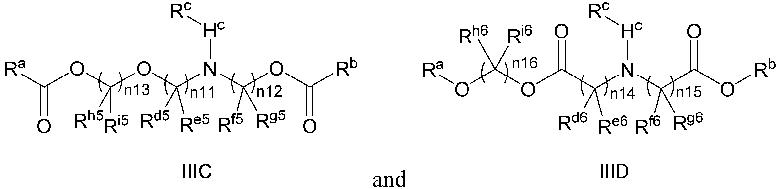
wherein, H^{c}, R^{a}, R^{b} and R^{c} are as defined in any embodiment of the compound of formula I;
R^{d3}, R^{d4}, R^{d5}, R^{d6}, R^{e3}, R^{e4}, R^{e5}, R^{e6}, R^{f3}, R^{f4}, R^{f5}, R^{f6}, R^{g3}, R^{g4}, R^{g5}, R^{g6}, R^{h3}, R^{h4}, R^{h5}, R^{h6}, Rⁱ³, Rⁱ⁴, Rⁱ⁵, Rⁱ⁶ are as defined in any embodiment of the compounds of formulas IC, ID, IE and IF;
n5, n6, n7, n8, n9, n10, n11, n12, n13, n14, n15 and n16 are as defined in any embodiment of the compounds of formulas IC, ID, IE and IF.

In some embodiments, the compound of formula III or a salt thereof is a compound with the following structure or a salt thereof,

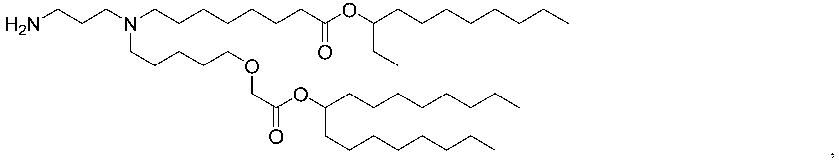

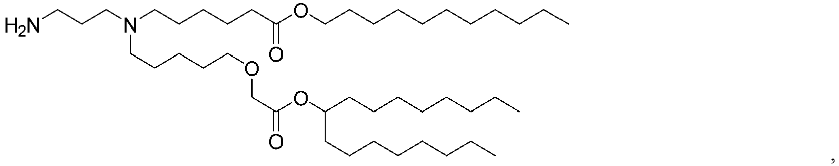

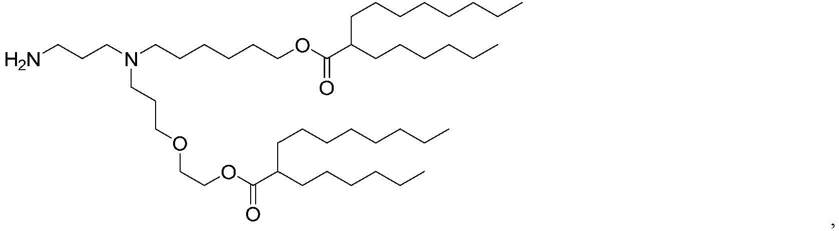

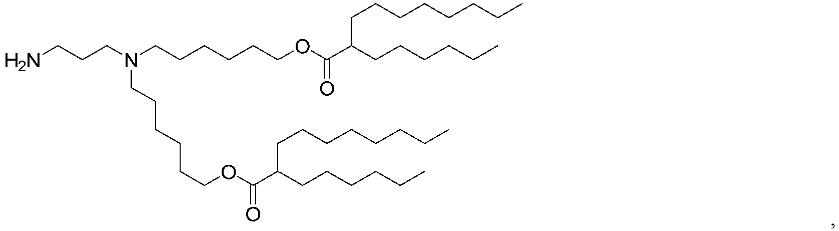

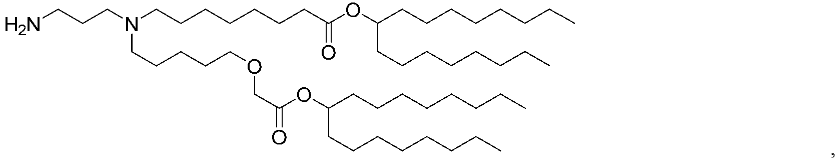

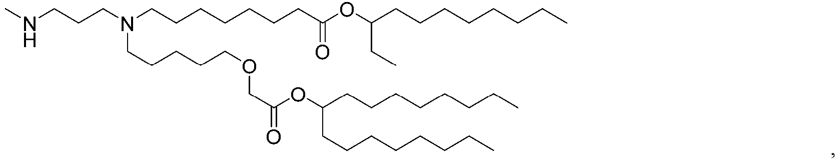

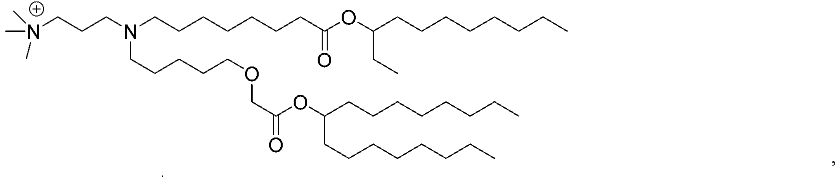

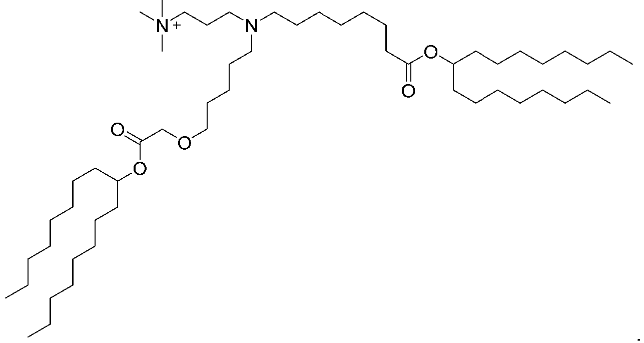

The present disclosure provides an isotope substitute of the above-described compound or a salt thereof, In some embodiments, the isotope substitute is a deuterated substitute.

The present disclosure provides a pharmaceutical composition comprising the above-described lipid nanoparticle composition or the above-described compound or a salt thereof or the above-described isotope substitute, and a pharmaceutically acceptable excipient.

In some embodiments, the above-described lipid nanoparticle composition, the above-described compound or a salt thereof or the above-described isotope substitute is present in a therapeutically effective amount.

In some embodiments, the pharmaceutical composition comprises 0.01% to 99.99% of a pharmaceutically acceptable excipient, based on the total weight of the pharmaceutical composition. In some embodiments, the pharmaceutical composition comprises 0.1% to 99.9% of a pharmaceutically acceptable excipient. In some embodiments, the pharmaceutical composition comprises 0.5% to 99.5% of a pharmaceutically acceptable excipient. In some embodiments, the pharmaceutical composition comprises 1% to 99% of a pharmaceutically acceptable excipient. In some embodiments, the pharmaceutical composition comprises 2% to 98% of a pharmaceutically acceptable excipient.

The present disclosure also provides use of the above-described lipid nanoparticle composition or the above-described compound or a salt thereof or the above-described isotope substitute or the above-described pharmaceutical composition in the preparation of a medicament for inducing an immune response in a subject.

The present disclosure also provides use of the above-described lipid nanoparticle composition or the above-described compound or a salt thereof or the above-described isotope substitute or the above-described pharmaceutical composition in the preparation of a medicament for preventing and/or treating a disease or disorder related to overexpression of polypeptide.

The present disclosure also provides use of the above-described lipid nanoparticle composition or the above-described compound or a salt thereof or the above-described isotope substitute or the above-described pharmaceutical composition in the preparation of a medicament for preventing and/or treating a disease or disorder related to underexpression of polypeptide.

In some embodiments, the disease or disorder includes, but is not limited to cancer, infection, autoimmune disease, neurodegenerative disease, and inflammation.

The present disclosure also provides use of the above-described lipid nanoparticle composition or the above-described compound or a salt thereof or the above-described isotope substitute or the above-described pharmaceutical composition in the preparation of a medicament for preventing and/or treating cancer, infection, autoimmune disease, neurodegenerative disease, and inflammation.

In another aspect, the present disclosure also provides a method for preventing and/or treating a disease or disorder related to inducing an immune response in a subject, comprising administering the above-described lipid nanoparticle composition or the above-described compound or a salt thereof or the above-described isotope substitute or the above-described pharmaceutical composition to a subject.

The present disclosure also provides a method for preventing and/or treating a disease or disorder related to overexpression of polypeptide, comprising administering the above-described lipid nanoparticle composition or the above-described compound or a salt thereof or the above-described isotope substitute or the above-described pharmaceutical composition to a subject.

The present disclosure also provides a method for preventing and/or treating a disease or disorder related to underexpression of polypeptide, comprising administering the above-described lipid nanoparticle composition or the above-described compound or a salt thereof or the above-described isotope substitute or the above-described pharmaceutical composition to a subject.

The present disclosure also provides a method for preventing and/or treating cancer, infection, autoimmune disease, neurodegenerative disease, and inflammation, comprising administering the above-described lipid nanoparticle composition or the above-described compound or a salt thereof or the above-described isotope substitute or the above-described pharmaceutical composition to a subject.

The present disclosure also provides the above-described lipid nanoparticle composition or the above-described compound or a salt thereof or the above-described isotope substitute or the above-described pharmaceutical composition for use in preventing and/or treating a disease or disorder related to inducing an immune response in a subject.

The present disclosure also provides the above-described lipid nanoparticle composition or the above-described compound or a salt thereof or the above-described isotope substitute or the above-described pharmaceutical composition for use in preventing and/or treating a disease or disorder related to overexpression of polypeptide.

The present disclosure also provides the above-described lipid nanoparticle composition or the above-described compound or a salt thereof or the above-described isotope substitute or the above-described pharmaceutical composition for use in preventing and/or treating a disease or disorder related to underexpression of polypeptide.

The present disclosure also provides the above-described lipid nanoparticle composition or the above-described compound or a salt thereof or the above-described isotope substitute or the above-described pharmaceutical composition for use in preventing and/or treating cancer, infection, autoimmune disease, neurodegenerative disease, and inflammation.

The present disclosure also provides use of the above-described compound of formula I or a salt thereof or the compound of formula III or a salt thereof in the preparation of a lipid nanoparticle composition targeting lung.

The lipid nanoparticle composition and compounds of the present disclosure have good targeting to lung.

### Definition of terms

The salts of compounds described in the present disclosure include "acid" addition salts and "base" addition salts. For example, the salts are formed by acid-base reactions of acids with basic groups (amino groups), and the acids include organic or inorganic acids.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. There may be additional asymmetric carbon atoms in substituents such as alkyl. All these isomers and mixtures thereof fall within the scope of the present disclosure. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formed from amines).

In the chemical structure of the compound of the present disclosure, the bond " 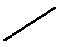 " represents an unspecified configuration, that is, if a chiral isomer exist in a chemical structure, the bond " 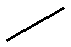 " may be " 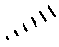 " or " 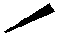 ", or contains both the configurations of " 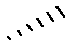 " and " 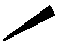 ". In the chemical structure of the compound of the present disclosure, the bond " 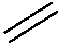 " represents an unspecified configuration, that is, it may be a Z or E configuration, or contains both the configurations.

Unless specified otherwise, the symbol 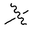 as used herein means it may be connected to one or more arbitrary groups.

The compounds and intermediates of the present disclosure may also exist in different tautomeric forms, and all such forms are included within the scope of the present disclosure. The term "tautomer" or "tautomeric form" refers to structural isomers with different energies that can be interconverted via a low energy barrier. For example, proton tautomers (also known as prototropic tautomers) include interconversions via proton migration, such as keto-enol and imine-enamine, lactam-lactimide isomerizations. An example of lactam-lactim equilibrium is present between A and B as shown below.

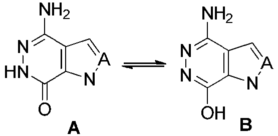

All tautomeric forms are within the scope of the present disclosure. The names of the compounds do not exclude any tautomers.

The present disclosure further includes isotopically-labeled compounds which are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl.

Unless otherwise specified, when a position is specifically assigned to deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is at least 1000 times, at least 2000 times, at least 3000 times, at least 4000 times, at least 5000 times, at least 6000 times, or higher times greater than the natural abundance of deuterium. The present disclosure further includes various deuterated forms of the compound of formula (I). Each available hydrogen atom connected to a carbon atom may be independently replaced by a deuterium atom. Those skilled in the art are able to synthesize the deuterated forms of the compound of formula (I) with reference to relevant literatures. In preparing the deuterated forms of the compound of formula (I), commercially available deuterated starting materials can be used, or they can be synthesized using conventional techniques with deuterated reagents including, but not limited to deuterated borane, a solution of tri-deuterated borane in tetrahydrofuran, deuterated lithium aluminum hydride, deuterated iodoethane, deuterated iodomethane and the like.

The term "optional" or "optionally" means that the event or circumstance described subsequently can, but need not occur, and such a description includes the situation in which the event or circumstance does or does not occur. For example, the expression "C₁₋₆ alkyl optionally substituted by halogen or cyano" means that halogen or cyano can be, but need not be present, and such a description includes the situation of the alkyl being substituted by halogen or cyano and the situation of the alkyl being not substituted by halogen or cyano.

The term "pharmaceutical composition" represents a mixture containing one or more compounds described herein or physiologically/pharmaceutically acceptable salts or prodrugs thereof and other chemical components, as well as other components such as physiologically/pharmaceutically acceptable carriers and excipients. The pharmaceutical composition is intended to promote administration to an organism and facilitate the absorption of the active ingredient and thus the exertion of its biological activity.

The term "pharmaceutical excipient" or "pharmaceutically acceptable excipient" includes, but is not limited to, any adjuvant, carrier, excipient, glidant, sweetener, diluent, preservative, dye/colorant, flavor enhancer, surfactant, wetting agent, dispersant, suspending agent, stabilizer, isotonic agent, solvent or emulsifier that has been approved by the U.S. Food and Drug Administration and is acceptable for use in humans or livestock animals.

The term "prevention" includes: (1) inhibiting onset of a disease in a subject or patient who may have a risk of acquiring the disease and/or be predisposed to the disease, but does not yet experience or exhibit any or all of pathologies or symptoms of the disease; and/or (2) slowing onset of pathologies or symptoms of a disease in a subject or patient who may have a risk of acquiring the disease and/or be predisposed to the disease, but does not yet experience or exhibit any or all of pathologies or symptoms of the disease.

The term "treatment" includes: (1) inhibiting a disease in a subject or patient who is experiencing or exhibiting a pathology or symptom of the disease (for example, preventing further development of the pathology and/or symptom), (2) ameliorating a disease in a subject or patient who is experiencing or exhibiting a pathology or symptom of the disease (for example, reversing the pathology and/or symptom), and/or (3) achieving any measurable alleviation of a disease in a subject or patient who is experiencing or exhibiting a pathology or symptom of the disease.

The term "effective amount", "effective dose", "effectively therapeutic amount" or "therapeutically effective amount" described in the present disclosure includes an amount sufficient to improve or prevent symptoms or conditions of a medical disease. The effective amount also means an amount sufficient to permit or facilitate diagnosis. The effective amount for specific patients or veterinary subjects may vary depending on factors such as the condition to be treated, the overall health status of the patient, the route and dosage of administration, and the severity of side effects. The effective amount can be the maximum dose or dosing regimen that avoids significant side effects or toxic effects.

As used herein, the term "object", "patient", "subject" or "individual" can be used interchangeably, including humans or non-human animals such as mammals, for example a human or monkey.

In the present disclosure, the term "comprise", "include" or "contain" may be replaced with "consist of".

The term "nucleic acid" refers to a polymer composed of nucleotides, for example deoxyribonucleotides (DNA) or ribonucleotides (RNA). The exemplary nucleic acid includes, but is not limited to deoxyribonucleotides (DNA) and ribonucleotides (RNA), including messenger RNA, RNAi-inducing agents, shRNA, siRNA, miRNA, antisense RNA and analogs.

The term "polypeptide", "peptide" and "protein" are used herein interchangeably, and refer to a polymer of amino acid residues. This term applies to amino acid polymers in which one or more amino acid residues are artificial chemical analogs of corresponding natural amino acids, and applies to natural amino acid polymers.

The term "alkyl" refers to a saturated aliphatic hydrocarbon group, which is a straight or branched chain group comprising 1 to 24 carbon atoms (C₁₋₂₄ alkyl). In some embodiments, the alkyl is a straight or branched chain group with 3 to 24 carbon atoms (C₃₋₂₄ alkyl). In some embodiments, the alkyl is a straight or branched chain group with 4 to 18 carbon atoms (C₄₋₁₈ alkyl). In some embodiments, the alkyl is a straight or branched chain group with 9 to 18 carbon atoms (C₉₋₁₈ alkyl). In some embodiments, the alkyl is a straight or branched chain group with 7 to 13 carbon atoms (C₇₋₁₃ alkyl). In some embodiments, the alkyl is a straight or branched chain group with 1 to 10 carbon atoms (C₁₋₁₀ alkyl). In some embodiments, the alkyl is a straight or branched chain group with 4 to 8 carbon atoms (C₄₋₈ alkyl). In some embodiments, the alkyl is a straight or branched chain group with 1 to 6 carbon atoms (C₁₋₆ alkyl). In some embodiments, the alkyl is a straight or branched chain group with 1 to 4 carbon atoms (C₁₋₄ alkyl). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof.

The alkyl can be unsubstituted or substituted by one or more groups selected from the group consisting of halogen, hydroxy, oxo, cyano, amino, C₁₋₆ alkyl, C₁₋₆ alkoxy, and 3 to 7 membered cycloalkyl.

The term "alkylene" refers to the remaining group after removing 1 hydrogen atom from the "alkyl" as defined above.

The term "alkenyl" refers to an unsaturated aliphatic hydrocarbon group containing at least one carbon-carbon double bond, including straight and branched alkenyl groups. In some embodiments, the alkenyl is a straight or branched alkenyl group with 2 to 24 carbon atoms (C₂₋₂₄ alkenyl). In some embodiments, the alkenyl is a straight or branched alkenyl group with 4 to 24 carbon atoms (C₄₋₂₄ alkenyl). In some embodiments, the alkenyl is a straight or branched alkenyl group with 4 to 18 carbon atoms (C₄₋₁₈ alkenyl). In some embodiments, the alkenyl is a straight or branched alkenyl group with 9 to 18 carbon atoms (C₉₋₁₈ alkenyl). In some embodiments, the alkenyl is a straight or branched alkenyl group with 7 to 13 carbon atoms (C₇₋₁₃ alkenyl). In some embodiments, the alkenyl is a straight or branched alkenyl group with 2 to 10 carbon atoms (C₂₋₁₀ alkenyl). In some embodiments, the alkenyl is a straight or branched alkenyl group with 4 to 10 carbon atoms (C₄₋₁₀ alkenyl). In some embodiments, the alkenyl is a straight or branched alkenyl group with 4 to 8 carbon atoms (C₄₋₈ alkenyl). Exemplary alkenyl groups include vinyl, propenyl, n-butenyl, isobutenyl, 3-methylbut-2-enyl, n-pentenyl, heptenyl, octenyl, cyclohexyl-butenyl, and decenyl.

The alkenyl can be unsubstituted or substituted by one or more groups selected from the group consisting of halogen, hydroxy, oxo, cyano, amino, C₁₋₆ alkyl, C₁₋₆ alkoxy, and 3 to 7 membered cycloalkyl.

The term "alkoxy" refers to an -O-alkyl group, in which the alkyl is as defined above.

The term "cycloalkyl" refers to a saturated or partially unsaturated monocyclic or polycyclic hydrocarbon group, in which the cycloalkyl ring contains 3 to 8 carbon atoms, preferably 3 to 7 carbon atoms. Non-limiting examples of the monocyclic cycloalkyl include cyclopropyl, cyclobutyl, cyclopentyl, cyclopentenyl, cyclohexyl, cyclohexenyl, cyclohexadienyl, etc. The polycyclic cycloalkyl includes spiro cycloalkyl, fused cycloalkyl and bridged cycloalkyl. The cycloalkyl can be unsubstituted or substituted by one or more groups selected from the group consisting of halogen, hydroxy, oxo, cyano, amino, C₁₋₆ alkyl, C₁₋₆ alkoxy, and 3 to 7 membered cycloalkyl.

The terms "branched alkyl" and "branched alkenyl" refer to alkyl and alkenyl groups in which the carbon chain has a non-terminal carbon atom connected to at least one other carbon atom, without forming a cyclic group such as spiro ring. As an example, C₆ alkyl includes: 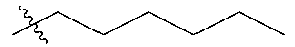 (straight), 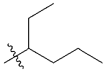 (branched) or 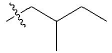 (branched).

The term "hydroxy" refers to an -OH group.

The term "halogen" refers to fluorine, chlorine, bromine or iodine.

The term "substituted" means that one or more hydrogen atoms in a group, preferably up to 5, and more preferably 1 to 3 hydrogen atoms in a group, are independently replaced by a corresponding number of substituents. It goes without saying that the substituents only exist in their possible chemical position. Those skilled in the art are able to determine whether the substitution is possible or impossible by experiments or theory without excessive efforts.

### DESCRIPTION OF THE DRAWINGS

Figure 1 shows the fluorescence intensity in various tissues of the mice injected with mRNA lipid nanoparticles via the tail vein. From left to right, each group is corresponding to heart, lung, liver, spleen, and kidney. In Figure 1, * indicates a statistically significant difference, P<0.05.
Figure 2 shows the proportions of protein expression in various tissues of the mice injected with mRNA lipid nanoparticles via the tail vein. From left to right, each group is corresponding to heart, lung, liver, spleen, and kidney.
Figure 3 shows the fluorescence intensity in various tissues of the mice injected intratracheally with mRNA lipid nanoparticles. From left to right, each group is corresponding to heart, lung, liver, spleen, and kidney. In Figure 3, * indicates a statistically significant difference, P<0.05.
Figure 4 shows the proportions of protein expression in various tissues of the mice injected intratracheally with mRNA lipid nanoparticles. From left to right, each group is corresponding to heart, lung, liver, spleen, and kidney.
Figure 5 shows the measured interleukin 6 (IL-6) concentrations in serum of the mice injected with mRNA lipid nanoparticles via the tail vein. In Figure 5, ** indicates a statistically significant difference, P<0.01.
Figure 6 shows the measured interleukin 6 (IL-6) concentrations in serum of the mice injected intratracheally with mRNA lipid nanoparticles. In Figure 6, ** indicates a statistically significant difference, P<0.01.
Figure 7 shows the encapsulation efficiency after nebulization of LNP. In the figure, *** indicates a statistically significant difference, P<0.001.
Figure 8 shows the relative protein expression levels of LNPs with different composition ratios in lung.
Figure 9 shows the fluorescence intensity in lung of the mice injected with mRNA lipid nanoparticles via the tail vein. In the figure, * indicates a statistically significant difference, P<0.05.

### DETAILED DESCRIPTION OF THE INVENTION

The present disclosure is further described below in combination with the examples, but these examples are not intended to limit the scope of the present disclosure.

The experimental methods without specifying specific conditions in the examples of the present disclosure are generally conducted under conventional conditions, or conditions recommended by the manufacturers of the starting materials or commercial products. The reagents without specifying specific sources are conventional reagents purchased from the market.

The structures of the compounds are identified by nuclear magnetic resonance (NMR) and/or mass spectrometry (MS). NMR shift (δ) is given in units of 10⁻⁶ (ppm). NMR is determined on a Bruker AVANCE-400 nuclear magnetic spectrometer. The solvents for determination are deuterated dimethyl sulfoxide (DMSO-*d₆*), deuterated chloroform (CDCl₃), and deuterated methanol (Methanol-*d₄*), and the internal standard is tetramethylsilane (TMS).

HPLC is determined on an Agilent1100 high pressure liquid chromatograph with a GAS15B DAD UV detector and Water Vbridge C18 150 × 4.6 mm 5 µM column.

MS is determined on an Agilent6120 triple quadrupole mass spectrometer with a G1315D DAD detector and a Waters Xbridge C18 4.6 × 50 mm, 5 µM column, scanning in a positive/negative ion mode with a mass scanning range from 80 to 1200.

Yantai Huanghai HSGF254 silica gel plates are used for thin-layer chromatography. For the gel plates used, the specification is 0.2 mm ± 0.03 mm for thin-layer chromatography (TLC), and 0.4 mm - 0.5 mm for separation and purification of products by thin-layer chromatography.

Combiflash Rf150 (TELEDYNE ISCO) or Isolara one (Biotage) is used as the rapid column purification system.

Yantai Huanghai 200~300 mesh silica gel or 300~400 mesh silica gel is generally used as a carrier for normal-phase column chromatography, or Changzhou Santai prefilled ultra-pure normal-phase silica gel columns (40-63 µm, 60 g, 24 g, 40 g, 120 g or other specifications) are used.

The known starting materials of the present disclosure can be synthesized by or according to the methods known in the art, or can be purchased from Shanghai Titan Technology, ABCR GmbH & Co. KG, Acros Organics, Aldrich Chemical Company, Accela ChemBio Inc, Bide Pharm and other companies.

Unless specifically stated in the examples, all reactions can be carried out under nitrogen atmosphere.

Nitrogen atmosphere means that a reaction flask is connected to a nitrogen balloon with a volume of about 1 L.

Hydrogen atmosphere means that a reaction flask is connected to a hydrogen balloon with a volume of about 1 L.

Hydrogen is produced by a hydrogen generator of the QPH-1L type from Shanghai Quan Pu Scientific Instruments.

The nitrogen atmosphere or hydrogen atmosphere is generally generated by vacuuming and filling with nitrogen or hydrogen, 3 times repeatedly.

Unless specifically stated in the examples, a solution refers to an aqueous solution.

Unless specifically stated in the examples, the reaction temperature is room temperature, which is 20°C to 30°C.

Reaction processes in the examples are monitored by thin-layer chromatography (TLC). The developing agents used for the reactions, the eluent systems of the column chromatography and the developing systems of the thin-layer chromatography used for purifying the compounds, and the volume ratios of the solvents are adjusted depending on the polarity of the compounds. A small amount of basic or acidic reagents such as triethylamine and acetic acid can also be added for adjustment.

### Example 1: Preparation of Compound I-1

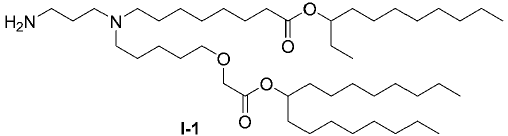

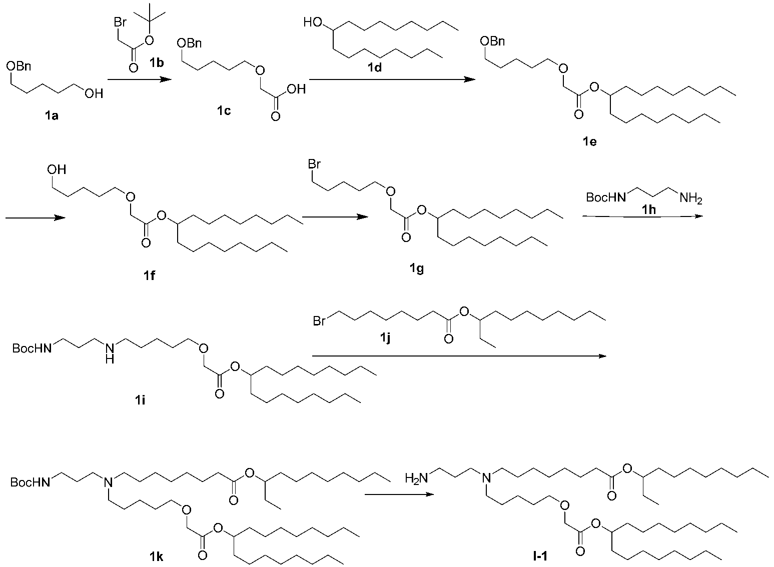

### Step 1:

Water (46.8 mL) was added to a 500 mL three-necked flask, cooled to 5°C, followed by the addition of sodium hydroxide (46.8 g, 1170 mmoL) during which the internal temperature was maintained below 15°C. After complete dissolution of sodium hydroxide, toluene (46.8 mL) and Compound 1a (9.71 g, 50.0 mmol, purchased from Adamas) were added and stirred at room temperature. After cooling to 5°C or lower again, Compound 1b (34.1 g, 175 mmol, purchased from Adamas) was added dropwise. The resulting mixture was heated to room temperature in an oil bath, and stirred until complete reaction. The reaction solution was cooled to 5°C or lower, followed by the addition of water (200 mL), and extracted with methyl tert-butyl ether (100 mL×3). The aqueous phase was cooled to 5°C or lower, followed by the addition of concentrated hydrochloric acid until the pH was about 3, and extracted with DCM (100 mL×5) again. The DCM phases were combined, washed with saturated brine, dried over anhydrous sodium sulfate and concentrated under reduced pressure. The residues were purified by column chromatography (EA/PE=0-100%) to obtain 11.97 g of Compound 1c (yield 94%).

¹H NMR (400 MHz, CDCl₃): *δ* 8.50 (brs, 1H), 7.36-7.26 (m, 5H), 4.51 (s, 2H), 4.13~4.10 (m,2H), 3.56 (t, 2H), 3.48 (t, 2H), 1.67~1.61 (m, 4H), 1.50~1.43 (m, 2H).

### Step 2:

Compound 1c (21.7 g, 86.0 mmol) was weighed and placed into a reaction flask, followed by the addition of DCM (434 mL), Compound 1d (19.9 g, 77.4 mmol, obtained according to the preparation method in the literature: Tetrahedron, 2011, vol. 67, # 2, p. 303 - 311), DMAP (10.5 g, 30.1 mmol) and EDCI (19.8 g, 103 mmoL) successively, and reacted at room temperature overnight. The complete reaction was monitored by TLC. Water (500 mL) was added to separate the reaction solution, and the aqueous phase was extracted with DCM (300 mL). The organic phases were combined, washed with saturated aqueous sodium bicarbonate solution and then with saturated brine, dried over anhydrous sodium sulfate and concentrated under reduced pressure,. The residues were purified by column chromatography (EA/PE=0-5%) to obtain 41.5 g of Compound 1e (yield 98%).

¹H NMR (400 MHz, CDCl₃): *δ* 7.34~7.33 (m, 4H), 7.28~7.27 (m, 1H), 4.97~4.95 (m, 1H), 4.50 (s, 2H), 4.04 (s, 2H), 3.54~3.46 (m, 4H), 1.69~1.62 (m, 4H) , 1.58~1.43 (m, 2H), 1.25 (s, 26H), 0.89~0.83 (m, 8H).

### Step 3:

Compound 1e (41.5 g, 84.3 mmol) was weighed and dissolved into THF (208 mL), followed by the addition of Pd(OH)₂/C (4.15 g, 20%). Hydrogen gas was introduced by a double-layer hydrogen balloon, and the reaction was stirred at room temperature for 5 hours. The complete reaction was monitored by TLC. The resulting mixture was subjected to suction filtration through diatomite, and the filter cake was washed with EA (100 mL×3). The filtrate was concentrated under reduced pressure, and purified by column chromatography (EA/PE=50%) to obtain 27.0 g of Compound 1f (yield 80%).

¹H NMR (400 MHz, CDCl₃): *δ* 4.98~4.93 (m, 1H), 4.05 (s, 2H), 3.66 (t, 2H), 3.54 (t, 2H), 1.70~1.58 (m, 4H) , 1.53~1.43(m, 4H), 1.25 (s, 26H), 0.89~0.86 (m, 6H).

### Step 4:

Compound 1f (6.01 g, 15.0 mmol) was weighed and dissolved into ultra dry DCM (60 mL), followed by the addition of carbon tetrabromide (7.46 g, 22.5 mmoL) in an ice bath, and stirred for 10 minutes. triphenylphosphine (5.51 g, 21.0 mmoL) was added and then stirred at room temperature until complete reaction. The reaction was purified by column chromatography (EA/PE=0-5%) to obtain 6.75 g of Compound 1g (yield 97%).

¹H NMR (400 MHz, CDCl₃): *δ* 4.99~4.93 (m, 1H), 4.05 (s, 2H), 3.54 (t, 2H), 3.41 (t, 2H), 1.94~1.86 (m, 2H), 1.70~1.63 (m, 2H), 1.58~1.52 (m, 5H), 1.26 (s, 25H), 0.88 (t, 6H).

### Step 5:

Compound 1g (1.0 g, 2.2 mmol) and Compound 1h (1.9 g, 11.0 mmol, purchased from Energy Chemical) were dissolved into 20 mL of anhydrous ethanol, and heated until complete reaction under nitrogen atmosphere. The reaction solution was cooled to room temperature, concentrated under reduced pressure, and purified by column chromatography (DCM:MeOH=10:1-5:1) to obtain 1.0 g of Compound 1i (yield: 83%).

¹H NMR (400 MHz, CDCl₃): *δ* 5.13 (br s 1H), 4.99~4.93 (m, 1H), 4.04 (s, 2H), 3.53 (t, 2H), 3.25~3.16 (m, 2H), 2.69 (t, 2H), 2.63 (t, 2H), 1.71~ 1.61 (m, 4H), 1.57~1.40 (m, 18H), 1.33~1.19 (m, 24H), 0.92~0.84 (m, 6H).

### Step 6:

Compound 1i (0.487 g, 0.87 mmol), Compound 1j (0.300 g, 0.795 mmol, obtained according to the preparation method in the patent WO2018/170306), potassium iodide (0.145 g, 0.87 mmol), potassium carbonate (0.438 g, 3.018 mmol) were added to a 25 mL flask, followed by the addition of 5 mL of cyclopentyl methyl ether and 5 mL of anhydrous acetonitrile. The mixture was heated until complete reaction under nitrogen atmosphere. The reaction was cooled to room temperature and filtered. The filtrate was concentrated, and purified by column chromatography (PE:EA=10:1) to obtain 0.5 g of Compound 1k (yield: 74%).

¹H NMR (400 MHz, CDCl₃): *δ* 5.60 (br s 1H), 4.99~4.93 (m, 1H), 4.84~4.78 (m, 1H), 4.04 (s, 2H), 3.53 (t, 2H), 3.21~3.12 (m, 2H), 2.51~2.25 (m, 8H), 1.67~ 1.25 (m, 71H), 0.89~0.83 (m, 12H).

### Step 7:

Compound 1k (0.5 g, 0.586 mmol) was dissolved into anhydrous dichloromethane (6.0 mL), followed by the addition of trifluoroacetic acid (2.0 mL) at room temperature. The mixture was reacted until almost completion and concentrated under reduced pressure. The residues were purified by column chromatography (water:acetonitrile=35%-95%) to obtain 370 mg of Compound I-1 (yield: 84%).

¹H NMR (400 MHz, CDCl₃): *δ* 4.99~4.93 (m, 1H), 4.84~4.78 (m, 1H), 4.04 (s, 2H), 3.51 (t, 2H), 2.72 (t, 2H), 2.46~2.35 (m, 6H), 2.29 (t, 2H), 1.67~ 1.25 (m, 62H), 0.89~0.83 (m, 12H).

### Example 2: Preparation of Compound I-2

It was prepared according to the preparation method in WO2018170306A1.

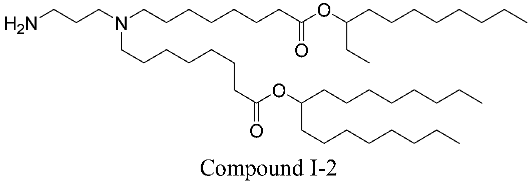

### Example 3: Preparation of Compound II-1

It was prepared according to the preparation method in WO2023125738A1.

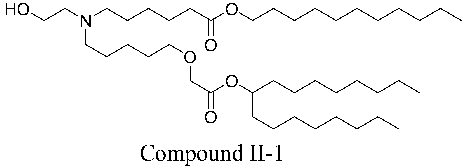

### Example 4: Preparation of Compound I-8

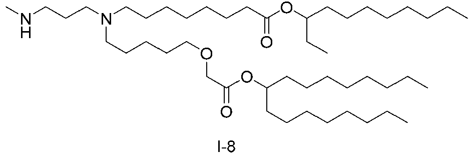

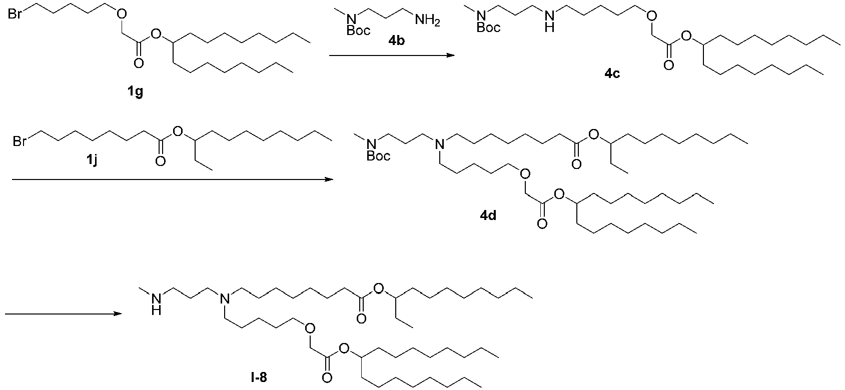

### Step 1:

Compounds **1g** (1.0 g, 2.2 mmol) and 4b(2.0 g, 10.8 mmol, purchased from Energy Chemical) were dissolved into 50 mL of anhydrous ethanol, and heated to an internal temperature of 60°C, and reacted for 16 hours. The heating was stopped, and the reaction solution was cooled to room temperature, and concentrated. The crude product was purified by silica gel column chromatography (DCM:MeOH=10:1-5:1) to obtain 4c (about 0.3 g, yield: 27%).

¹H NMR (400 MHz, CDCl₃): *δ* 4.92~ 4.96 (m, 1H), 4.03 (s, 2H), 3.51~ 3.54 (m, 2H), 3.39~ 3.42 (m, 2H), 2.84~ 2.93 (m, 7H), 2.17~ 2.29 (m, 2H),1.88~ 1.92 (m, 2H), 1.25~ 1.70 (m, 42H), 0.86~ 0.89 (m, 6H).

### Step 2:

4c (0.23 g, 0.40 mmol), 1j(0.15 g, 0.40 mmol), potassium iodide (0.068 g, 0.40 mmoL), and potassium carbonate (0.28 g, 2.01 mmol) were added to a 25 mL flask, followed by the addition of 5 mL of cyclopentyl methyl ether and 5 mL of anhydrous acetonitrile. The mixture was heated to an external temperature of 86°C, and reacted for 16 hours. The heating was stopped, and the reaction solution was cooled to room temperature, filtered, and concentrated. The crude product was purified by column chromatography (PE:EA=10:1) to obtain 4d (about 0.1 g, yield: 28%).

¹H NMR (400 MHz, CDCl₃): *δ* 4.94~ 4.97 (m, 1H), 4.79~ 4.82 (m, 1H),4.13 (s, 2H), 3.50~ 3.53 (m, 2H), 3.39~ 3.42 (m, 2H), 3.20 (s, 2H), 2.84 (s, 3H), 2.20~ 2.40 (m, 8H), 1.25~ 1.70 (m, 69H), 0.86~ 0.89 (m, 12H).

### Step 3:

Compound 4d (0.1 g, 0.12 mmol) was dissolved into 4.0 mL of anhydrous dichloromethane, followed by the addition of 1.0 mL of trifluoroacetic acid at room temperature. The mixture was reacted at room temperature for 4 hours. The complete reaction was monitored by LC/MS. The reaction solution was subjected to concentration as post-treatment, and the crude product was purified by ODS reversed-phase chromatography (water:acetonitrile=35%-95%) to obtain **I-8** (about 80.3 mg, yield: 79%).

MS (ESI): m/z 767.6 [M-CF₃COOH+H]⁺.

¹H NMR (400 MHz, CDCl₃): *δ* 4.90~ 4.94 (m, 1H), 4.78~ 4.82 (m, 1H),4.03 (s, 2H), 3.52~ 3.55 (m, 2H), 3.39~ 3.42 (m, 2H), 3.05~ 3.08 (m, 8H), 2.74 (s, 3H), 2.27~ 2.31 (m, 4H), 1.25~ 1.77 (m, 59H), 0.86~ 0.89 (m, 12H).

### Example 5: Preparation of Compound I-10

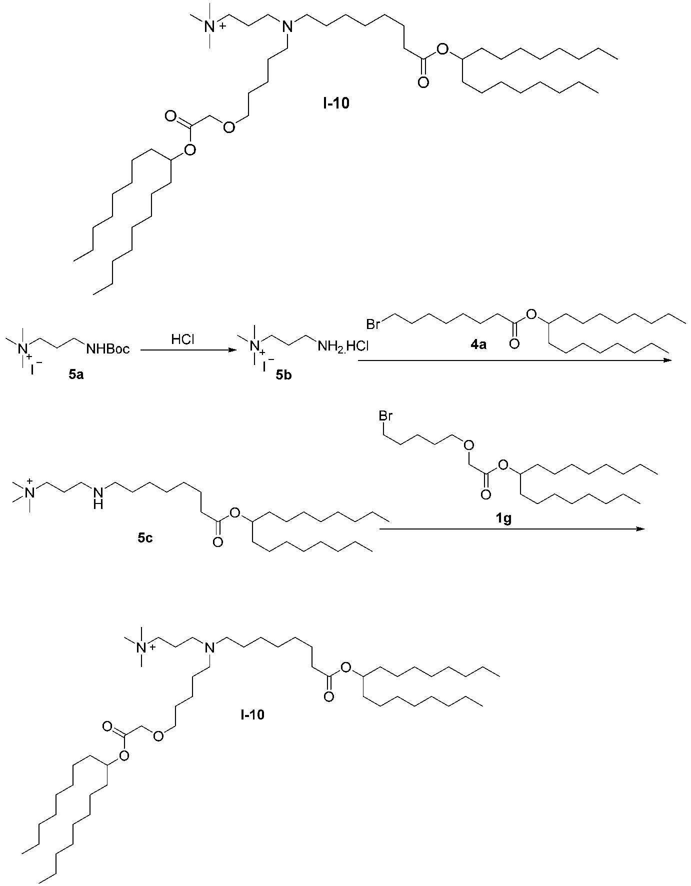

### Step 1:

Compound 5a (2.0 g, 9.2 mmol, obtained using the preparation method in the patent US2003/235916) was added to a flask, followed by the addition of anhydrous DCM (5 mL), and stirred for dissolution, followed by the addition of 4 M HCl/dioxane solution (11.5 mL) at room temperature. The mixture was reacted until complete reaction, and concentrated under reduced pressure to obtain 1.6 g of a crude product, which was directly used for the next step.

¹H NMR (400 MHz, DMSO): *δ* 8.24 (br s, 3H), 3.45~3.44 (m, 2H), 3.08 (s, 9H), 2.88~2.83 (m, 2H), 2.07~1.99 (m, 2H).

### Step 2:

Compound 5b (197 mg, 0.70 mmol) and Compound 4a (65 mg, 0.14 mmol) were added to a flask, followed by the addition of anhydrous ethanol (5 mL), and stirred for dissolution, followed by the addition of sodium carbonate (149 mg, 1.41 mmoL). The mixture was heated until complete reaction under nitrogen atmosphere. The reaction was filtered and concentrated. The residues were purified by column chromatography (acetonitrile:methanol=0~100%) to obtain 70 mg of Compound 5c (yield: 80%).

¹H NMR (400 MHz, CDCl₃): *δ* 4.89~4.51 (m, 1H), 3.68~3.46 (m, 3H), 3.36~3.16 (m, 10H), 2.31~2.21 (m, 2H), 1.98~1.78 (m, 2H), 1.63~1.17 (m, 41H), 0.92~0.82 (m, 6H).

### Step 3:

Compound 5c (340 mg, 0.54 mmol) and Compound 1g (252 mg, 0.54 mmol) were added to a flask, followed by the addition of potassium carbonate (375.5 g, 2.72 mmol), potassium iodide (99.4 mg, 0.6 mmoL), and solvents (5 mL of anhydrous acetonitrile and 5 mL of cyclopentyl methyl ether). The mixture was heated until complete reaction under nitrogen atmosphere and cooled to room temperature. The reaction was filtered and concentrated. The residues were purified by column chromatography (acetonitrile: methanol=0~100%) to obtain 390 mg of Compound I-10 (yield: 81%).

¹H NMR (400 MHz, MeOD): *δ* 4.99~4.92 (m, 1H), 4.88~4.83 (m, 1H), 4.08 (s, 2H), 3.56 (t, 2H), 3.4~3.40 (m, 2H), 3.23~3.19 (m, 14H), 2.33~2.22 (m, 4H), 1.85~1.53 (m, 18H), 1.41~1.29 (m, 55H), 0.91~0.88 (m, 12H).

### Example 6: Preparation of lipid nanoparticle compositions

### 1.1. Preparation method

Compound I-1, Compound I-2, Compound II-1, Compound I-8, Compound I-10, and DOTAP (AVT company) were dissolved in an ethanol solution (0.2 mg/ml) respectively, and mixed with solutions of DSPC, cholesterol, and DMG-PEG (AVT company) in ethanol at the molar percentages in Table 1 respectively, to obtain lipid ethanol solutions. Luciferase-expressing mRNA (Luciferase, GenBank: MN728548.1) was dissolved in a 50 mM acetate buffer (pH 5) to prepare an aqueous mRNA solution (0.2 mg/mL). The lipid ethanol solution and the aqueous mRNA solution were mixed by microfluidics to prepare liposomes, wherein the volume ratio of ethanol phase to aqueous phase was 1:3, and the weight ratio of total lipids to mRNA was approximately 20:1. The ethanol was removed by dialysis against a 20 mM Tris solution (pH 7.5), and finally exchanged into a 8% sucrose solution containing 20 mM Tris (pH 7.5), to obtain a lipid nanoparticle composition in which the mRNA was encapsulated.

**Table 1**

| No. | Composition | mol% |
|---|---|---|
| LNP-1 | Compound I-1: DSPC: Cholesterol: DMG-PEG | 50:10:38.5:1.5 |
| LNP-2 | Compound I-1: Compound II-1: DSPC: Cholesterol: DMG-PEG | 25:25:10:38.5:1.5 |
| LNP-3 | Compound I-1: Compound II-1: DSPC: Cholesterol: DMG-PEG | 50:25:5:18.5:1.5 |
| LNP-4 | DOTAP: Compound II-1: DSPC: Cholesterol: DMG-PEG | 50:25:5:18.5:1.5 |
| LNP-5 | Compound I-2: DSPC: Cholesterol: DMG-PEG | 50:10:38.5:1.5 |
| LNP-6 | Compound I-2: Compound II-1: DSPC: Cholesterol: DMG-PEG | 50:25:5:18.5:1.5 |
| LNP-7 | Compound I-2: Compound II-1: DSPC: Cholesterol: DMG-PEG | 25:25:10:38.5:1.5 |
| LNP-8 | Compound II-1: DSPC: Cholesterol: DMG-PEG | 50:10:38.5:1.5 |
| LNP-9 | Compound I-8: Compound II-1: DSPC: Cholesterol: DMG-PEG | 50:25:5:18.5:1.5 |
| LNP-10 | Compound I-10: Compound II-1: DSPC: Cholesterol: DMG-PEG | 50:25:5:18.5:1.5 |

### 1.2. Characterization of the lipid nanoparticle compositions

The size and polydispersity index PDI of the lipid nanoparticles were measured using a Malvern Zetasizer Pro in a 173° backscatter detection mode with dynamic light scattering.

The encapsulation efficiency was determined using a Quant-iT RiboGreen RNA Assay Kit for the quantitative detection of RNA.

The pKa of cations in the liposomal nanoparticles was determined using a fluorescence analysis based on 6-(p-toluidino)-2-naphthalenesulfonic acid sodium salt (TNS). 150 mM NaCl, 10 mM sodium phosphate, 10 mM sodium citrate, 10 mM sodium borate, and buffers of various pH levels ranging from 3 to 11.5 were prepared. A 300 µM TNS solution was prepared and added to the buffers. The lipid nanoparticles were added to the buffers of different pH respectively and mixed thoroughly. Then, the fluorescence intensity at an excitation wavelength of 325 nm and an emission wavelength of 435 nm was measured at room temperature using a fluorescence microplate reader. A fitting analysis of the fluorescence data was performed, with the pKa being the pH value at which half-maximal fluorescence intensity was produced.

**Table 2**

| No. | Size (nm) | PDI | Encapsulation efficiency (%) | pKa |
|---|---|---|---|---|
| LNP-1 | 67.3 | 0.20 | 99.9 | 8.12 |
| LNP-2 | 157.0 | 0.27 | 99.5 | / |
| LNP-3 | 62.1 | 0.20 | 99.9 | 7.84 |
| LNP-4 | 79.8 | 0.12 | 97.0 | 10.23 |
| LNP-5 | 66.7 | 0.24 | 99.7 | / |
| LNP-6 | 58.9 | 0.17 | 99.6 | / |
| LNP-8 | 95.5 | 0.12 | 96.0 | 6.65 |
| LNP-9 | 75.4 | 0.17 | 99.2 | 7.87 |
| LNP-10 | 118.7 | 0.12 | 97.3 | 7.66 |

### Test Example 1: Evaluation of mRNA delivery efficiency and tissue distribution of the lipid nanoparticle compositions via tail vein injection in vivo

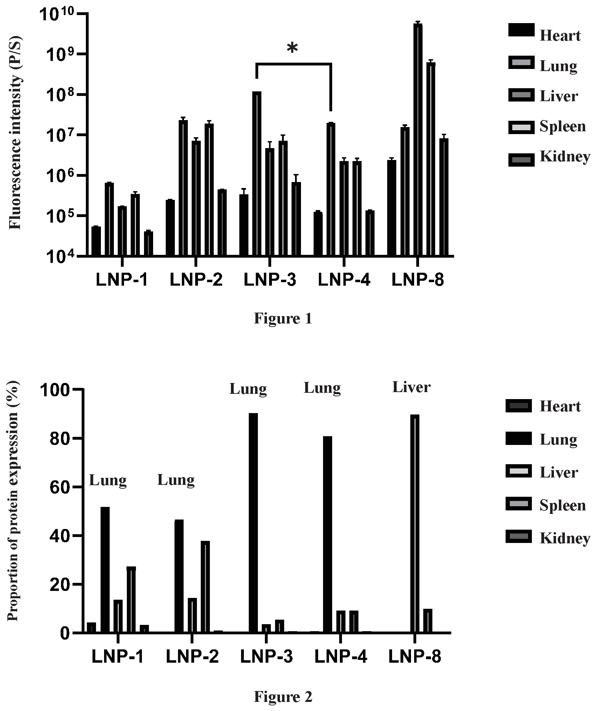

The lipid nanoparticle compositions encapsulating mRNA expressing luciferase (Luciferase, GenBank: MN728548.1) were injected into female C57 BL/6 mice 6-8 week old (purchased from Shanghai Model Organisms Center, Inc.) via the tail vein at a dose of 0.5 mg/kg. At 6 hours after administration, the luciferase substrate was injected intraperitoneally into each mouse, and the mouse was dissected to collect heart, liver, spleen, lung, and kidney. Fluorescence images of the tissues were captured using an IVIS small animal optical *in vivo* imaging system (PerkinElme), and the fluorescence intensity of each tissue was recorded. The level of fluorescence intensity represents the expression level of luciferase protein, i.e., it reflects the *in vivo* mRNA delivery efficiency of the lipid nanoparticle composition and the distribution thereof in various organic tissues. For the lipid nanoparticles to which each compound corresponded, the mean fluorescence intensity was calculated for at least three biological replicates. The data are shown in Table 3 and Figure 1. In FIG. 1, the fluorescence intensity refers to the fluorescence intensity of each tissue in the mice recorded using the IVIS small animal optical *in vivo* imaging system. The level of fluorescence intensity represents the expression level of luciferase protein, i.e., the level of *in vivo* mRNA delivery efficiency of the lipid nanoparticle composition and the tissue distribution thereof.

With LNP-4 and LNP-8 as controls, the mRNA delivery efficiency and tissue distribution were measured for the lipid nanoparticle compositions to which Composition LNP-1 containing Compound I-1 and Compositions LNP-2 and LNP-3 containing Compounds I-1 and II-1 corresponded, and which were injected into the mice via the tail vein.

**Table 3**

| No. | Fluorescence intensity (p/s) | | | | |
|---|---|---|---|---|---|
| | Heart | Lung | Liver | Spleen | Kidney |
| LNP-1 | 5.42E+04 | 6.53E+05 | 1.72E+05 | 3.44E+05 | 4.05E+04 |
| LNP-2 | 2.46E+05 | 2.31E+07 | 7.13E+06 | 1.87E+07 | 4.47E+05 |
| LNP-3 | 3.40E+05 | 1.20E+08 | 4.68E+06 | 7.15E+06 | 6.81E+05 |
| LNP-4 | 1.25E+05 | 2.00E+07 | 2.25E+06 | 2.23E+06 | 1.34E+05 |
| LNP-8 | 2.36E+06 | 1.56E+07 | 5.67E+09 | 6.24E+08 | 8.30E+06 |

**Table 4**

| No. | Fluorescence intensity proportion (%) | | | | |
|---|---|---|---|---|---|
| | Heart | Lung | Liver | Spleen | Kidney |
| LNP-1 | 4.29 | 51.7 | 13.58 | 27.23 | 3.21 |
| LNP-2 | 0.49 | 46.55 | 14.37 | 37.68 | 0.90 |
| LNP-3 | 0.26 | 90.32 | 3.52 | 5.39 | 0.51 |
| LNP-4 | 0.51 | 80.82 | 9.1 | 9.03 | 0.54 |
| LNP-8 | 0.1 | 0.3 | 89.6 | 9.9 | 0.1 |

Conclusion: as shown in Table 3 and Figure 1, the lipid nanoparticle compositions LNP 1 to 3 composed of Compound I-1 exhibited the highest fluorescence intensity in lung, i.e., the highest expression level of luciferase protein in lung. Compared with conventional lipid nanoparticle compositions such as siRNA-lipid nanoparticles that have already been on the market and deliver siRNA to the liver, the lipid nanoparticle compositions containing Compound I-1 delivers more nucleic acid drug to lung. The protein expression level of the lipid nanoparticle composition LNP-3 composed of Compound I-1 in lung was approximately 10 times higher than that of the lipid nanoparticle composition LNP-4 composed of DOTAP.

As shown in Table 4 and Figure 2, the proportion of fluorescence intensity represented the proportion of protein expression in each organ tissue and indicated that, the proportions of the lipid nanoparticle compositions LNP 1 to 3 composed of Compound I-1 in lung were all higher than those in liver and spleen, with the proportion of LNP-3 in lung exceeding 90%. The proportion of protein expression of the lipid nanoparticle composition LNP-3 containing Compound I-1 in lung was higher than that of the lipid nanoparticle composition LNP-4 containing DOTAP. The lipid nanoparticle composition LNP-8 of Compound II-1 was primarily distributed in liver, and distributed less than 1% in lung.

### Test Example 2: Evaluation of mRNA delivery efficiency and tissue distribution of the lipid nanoparticle compositions via intratracheal administration

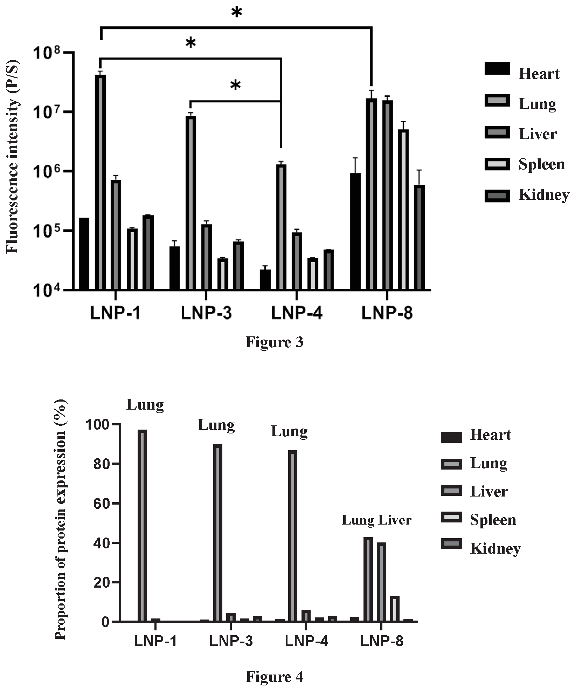

The lipid nanoparticle compositions encapsulating mRNA expressing luciferase were administered intratracheally into female C57 BL/6 mice 6-8 week old (purchased from Shanghai Model Organisms Center, Inc.) at a dose of 0.5 mg/kg. At 6 hours after administration, the luciferase substrate was injected intraperitoneally into each mouse, and the mouse was dissected to collect heart, liver, spleen, lung, and kidney. Fluorescence images of the tissues were captured using an IVIS small animal optical *in vivo* imaging system (PerkinElme), and the fluorescence intensity of each tissue was recorded. The level of fluorescence intensity represents the expression level of luciferase protein, i.e., it reflects the *in vivo* mRNA delivery efficiency of the lipid nanoparticle composition and the distribution thereof in various organic tissues. For the lipid nanoparticle compositions of each compound, the mean fluorescence intensity was calculated for at least three biological replicates. The data are shown in Table 5. The percentage of the mean fluorescence intensity of each tissue to the total fluorescence intensity was recorded to evaluate the tissue distribution of protein expression of the lipid nanoparticle compositions, as shown in Table 6. In FIG. 3, the fluorescence intensity refers to the fluorescence intensity of each tissue in the mice recorded using the IVIS small animal optical *in vivo* imaging system. The level of fluorescence intensity represents the expression level of luciferase protein, i.e., the level of *in vivo* mRNA delivery efficiency of the lipid nanoparticle composition and the tissue distribution thereof.

With the lipid nanoparticle composition containing DOTAP (LNP-4) as a control, the *in vivo* protein expression level and tissue distribution were measured for the lipid nanoparticle compositions LNP-1 and LNP-2 containing Compound I-1 administrated to the mice via the respiratory tract.

**Table 5**

| No. | Fluorescence intensity (p/s) | | | | |
|---|---|---|---|---|---|
| | Heart | Lung | Liver | Spleen | Kidney |
| LNP-1 | 1.64E+05 | 4.24E+07 | 7.26E+05 | 1.09E+05 | 1.83E+05 |
| LNP-3 | 5.53E+04 | 5.63E+06 | 1.29E+05 | 3.44E+04 | 6.70E+04 |
| LNP-4 | 2.22E+04 | 1.31E+06 | 9.34E+04 | 3.49E+04 | 4.80E+04 |
| LNP-8 | 9.30E+05 | 1.68E+07 | 1.58E+07 | 5.16E+06 | 6.01E+05 |

**Table 6**

| No. | Fluorescence intensity proportion (%) | | | | |
|---|---|---|---|---|---|
| | Heart | Lung | Liver | Spleen | Kidney |
| LNP-1 | 0.4 | 97.3 | 1.7 | 0.2 | 0.4 |
| LNP-3 | 1.1 | 89.8 | 4.6 | 1.6 | 2.9 |
| LNP-4 | 1.5 | 86.8 | 6.2 | 2.3 | 3.2 |
| LNP-8 | 2.4 | 42.9 | 40.1 | 13.1 | 1.5 |

Conclusion: as shown in Table 5 and Figure 3, the lipid nanoparticle compositions LNP-1 and LNP-3 containing Compound I-1 exhibited the highest fluorescence intensity in lung, i.e., the highest expression level of luciferase protein in lung. The protein expression level of the lipid nanoparticle composition LNP-1 containing Compound I-1 in lung was approximately 32 times higher than that of the lipid nanoparticle composition LNP-4 containing DOTAP.

As shown in Table 6 and Figure 4, the proportion of fluorescence intensity represented the proportion of protein expression in each organ tissue and indicated that, the proportions of the lipid nanoparticle compositions LNP-1 and LNP-3 containing Compound I-1 in lung were all higher than those in liver and spleen, with the proportion of LNP-1 in lung exceeding 90%. The proportions of protein expression of the lipid nanoparticle compositions LNP-1 and LNP-3 containing Compound I-1 in lung were higher than that of the lipid nanoparticle composition LNP-4 containing DOTAP. The fluorescence intensity and the proportion of fluorescence intensity of LNP-1 in lung were all higher than those of LNP-8. However, the fluorescence intensity of LNP-8 in liver was far higher than those of LNP-1 and LNP-3. This indicated that Compound I-1 in LNP is expressed more in lung and has better targeting.

### Test Example 3: Evaluation of in vivo safety of the lipid nanoparticle compositions

When exogenous substances enter the bodies of mammals, they induce an innate immune response, thereby promoting the production of cytokines. These exogenous substances can cause inflammatory reactions after entry into the body, which easily give rise to adverse reactions such as fever and edema in the body. The immunogenicity of the lipid nanoparticle compositions in body was therefore evaluated by evaluating the concentration of a cytokine such as interleukin 6 (IL-6) in the blood of mice injected with the lipid nanoparticle compositions. Lower cytokine concentrations indicate that the lipid nanoparticle compositions have lower immunogenicity, i.e., better biological safety.

The lipid nanoparticle compositions encapsulating mRNA expressing luciferase reporter gene were injected into female BALB/c mice 6-8 week old (purchased from Shanghai Model Organisms Center, Inc.) via the tail vein at a dose of 0.5 mg/kg, and after 6 hours, the blood was collected to isolate serum. The concentration of IL-6 in serum was measured using a mouse IL-6 ELISA kit. For the lipid nanoparticle compositions of each compound, the concentration of IL-6 in serum was measured for at least three biological replicates, and the mean IL-6 concentration was calculated.

**Table 7**

| No. | Serum IL-6 concentration (pg/mL) |
|---|---|
| LNP-3 | 492.6 |
| LNP-4 | 1712.8 |

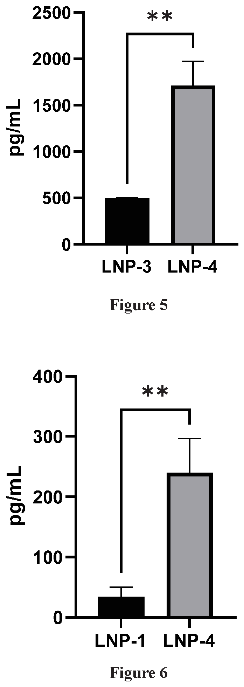

Conclusion: as shown in Table 7 and Figure 5, the IL-6 concentration in the mouse serum for the lipid nanoparticle composition LNP-3 was significantly lower than that of the comparative lipid nanoparticle composition LNP-4. This indicated that the lipid nanoparticle composition containing Compound I-1 exhibited lower immunogenicity and better biological safety *in vivo* than the lipid nanoparticle composition of the comparative compound DOTAP.

The lipid nanoparticle compositions encapsulating mRNA expressing luciferase reporter gene were injected intratracheally into female BALB/c mice 6-8 week old at a dose of 0.25 mg/kg, and after 6 hours, the blood was collected to isolate serum. The concentration of IL-6 in serum was measured using a mouse IL-6 ELISA kit. For the lipid nanoparticle compositions of each compound, the concentration of IL-6 in serum was measured for at least three biological replicates, and the mean IL-6 concentration was calculated.

**Table 8**

| No. | Serum IL-6 concentration (pg/mL) |
|---|---|
| LNP-1 | 34.0 |
| LNP-4 | 239.6 |

Conclusion: as shown in Table 8 and Figure 6, the IL-6 concentration in the mouse serum for the lipid nanoparticle composition LNP-1 was significantly lower than that of the comparative lipid nanoparticle composition LNP-4. This indicated that the lipid nanoparticle composition containing Compound I-1 exhibited lower immunogenicity and better biological safety *in vivo* than the lipid nanoparticle composition of the comparative compound DOTAP.

### Test Example 4: Evaluation of a characterization parameter of the lipid nanoparticle compositions after nebulization

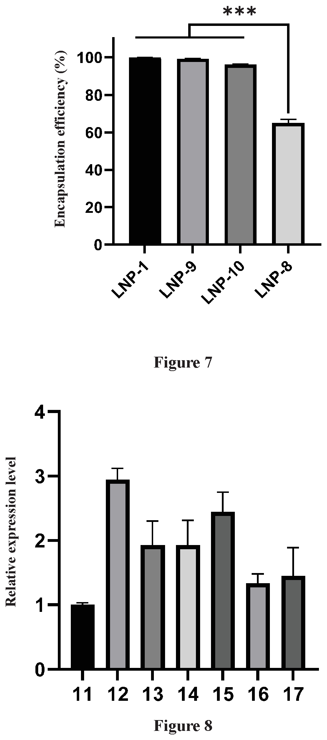

As an example, LNP-1 and LNP-8 were collected after nebulization by a vibrating mesh nebulizer (Aerogen-Solo), and the encapsulation efficiency of the lipid nanoparticle compositions was measured. The results are shown in Table 8 and Figure 7. After nebulization by the nebulizer, the lipid nanoparticle composition LNP-8 of Compound II-1 had a damaged structure under the influence of shear force during nebulization, causing a significant decrease in the encapsulation efficiency. However, LNP-1, LNP-9, and LNP-10 were able to withstand the influence of shear force during nebulization, and maintained consistent encapsulation efficiency before and after nebulization. The lipid nanoparticle compositions to which of Compound I-1, Compound I-8 and Compound I-10 are therefore suitable for nebulized inhalation administration for pulmonary drug delivery.

**Table 9**

| No. | Encapsulation efficiency before nebulization (%) | Encapsulation efficiency after nebulization (%) |
|---|---|---|
| LNP-1 | 99.9 | 99.9 |
| LNP-8 | 96.0 | 65 |
| LNP-9 | 99.2 | 99.1 |
| LNP-10 | 97.3 | 96.0 |

### Test Example 5: Evaluation of protein expression levels after delivering mRNA by the lipid nanoparticle compositions via intratracheal administration

According to the preparation and test methods of Example 6, the lipid nanoparticle compositions were prepared by mixing at the molar percentages in Table 10 and characterized. The prepared lipid nanoparticles were as shown in Tables 10 and 11.

The lipid nanoparticle compositions encapsulating human hepatocyte growth factor (NCBI: NM_000601.6) were administered intratracheally into female C57 BL/6 mice 6-8 week old (purchased from Shanghai Model Organisms Center, Inc.) at a dose of 0.5 mg/kg. At 24 hours after administration, the mouse was dissected to collect lung and measure the expression level of hepatocyte growth factor protein. The mean protein expression level was calculated for at least three biological replicates for each lipid nanoparticle composition. The relative protein expression levels of LNP-11 to LNP-17 were recorded with the mean protein expression level of LNP-11 set as 1 unit. Taking LNP-12 as an example, the relative protein expression level of LNP-12 = the protein expression level of LNP-12 / the protein expression level of LNP-11.

**Table 10**

| No. | Composition | mol% |
|---|---|---|
| LNP-11 | Compound I-1: Compound II-1:DSPC: Cholesterol: DMG-PEG | 0:48:10:40.5:1.5 |
| LNP-12 | Compound I-1: Compound II-1:DSPC: Cholesterol: DMG-PEG | 11.25:43.75:5:38.5:1.5 |
| LNP-13 | Compound I-1: Compound II-1: DSPC: Cholesterol: DMG-PEG | 12:36:10:40.5:1.5 |
| LNP-14 | Compound I-1: Compound II-1: DSPC: Cholesterol: DMG-PEG | 17.5:37.5:5:38.5:1.5 |
| LNP-15 | Compound I-1: Compound II-1: DSPC: Cholesterol: DMG-PEG | 24:24:10:40.5:1.5 |
| LNP-16 | Compound I-1: Compound II-1: DSPC: Cholesterol: DMG-PEG | 25:30:5:38.5:1.5 |
| LNP-17 | Compound I-1: Compound II-1: DSPC: Cholesterol: DMG-PEG | 36:12:10:40.5:1.5 |

**Table 11**

| No. | Size (nm) | PDI | Encapsulation efficiency (%) |
|---|---|---|---|
| LNP-11 | 70.8 | 0.11 | 98.1 |
| LNP-12 | 78.9 | 0.29 | 99.8 |
| LNP-13 | 60.3 | 0.30 | 99.9 |
| LNP-14 | 66.8 | 0.30 | 99.8 |
| LNP-15 | 61.7 | 0.23 | 99.9 |
| LNP-16 | 65.3 | 0.30 | 99.9 |
| LNP-17 | 53.9 | 0.28 | 99.9 |

**Table 12**

| No. | Mean relative protein expression level |
|---|---|
| LNP-11 | 1 |
| LNP-12 | 2.9 |
| LNP-13 | 1.9 |
| LNP-14 | 1.9 |
| LNP-15 | 2.4 |
| LNP-16 | 1.3 |
| LNP-17 | 1.4 |

As shown in Table 12 and Figure 8, the protein expression levels of the lipid nanoparticle compositions containing Compound I-1 were all higher than that of the lipid nanoparticle composition of Compound II-1. The lipid nanoparticle compositions LNP-12 and LNP-15 exhibited the highest protein expression levels.

### Test Example 6: Evaluation of mRNA delivery efficiency and tissue distribution of the lipid nanoparticle compositions via tail vein injection in vivo

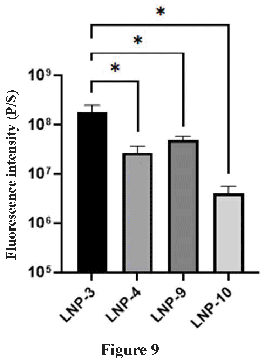

The lipid nanoparticle compositions encapsulating mRNA expressing luciferase (Luciferase, GenBank: MN728548.1) were injected into female C57 BL/6 mice 6-8 week old (purchased from Shanghai Model Organisms Center, Inc.) via the tail vein at a dose of 0.5 mg/kg. At 6 hours after administration, the luciferase substrate (D-luciferin potassium salt abs42075819) was injected intraperitoneally into each mouse, and the mouse was dissected to collect lung and liver. Fluorescence images of the tissues were captured using an IVIS small animal optical *in vivo* imaging system (PerkinElme), and the fluorescence intensity of each tissue was recorded. The level of fluorescence intensity represents the expression level of luciferase protein, i.e., it reflects the *in vivo* mRNA delivery efficiency of the lipid nanoparticle composition and the distribution thereof in various organic tissues. For the lipid nanoparticles of each compound, the mean fluorescence intensity thereof was calculated for at least three biological replicates. The data are shown in Table 13 and Figure 9. In FIG. 9, the fluorescence intensity refers to the fluorescence intensity of each tissue in the mice recorded using the IVIS small animal optical *in vivo* imaging system. The level of fluorescence intensity represents the expression level of luciferase protein, i.e., the level of *in vivo* mRNA delivery efficiency of the lipid nanoparticle composition and the tissue distribution thereof.

With LNP-4 as a control, the mRNA delivery efficiency and tissue distribution were measured for the lipid nanoparticle compositions of Composition LNP-3 containing Compounds I-1 and II-1, Composition LNP-9 containing Compounds I-8 and II-1, and Composition LNP-10 containing Compounds I-10 and II-1, and which were injected into the mice via the tail vein.

**Table 13**

| No. | Lung Fluorescence intensity (p/s) | Liver Fluorescence intensity (p/s) | Lung/Liver |
|---|---|---|---|
| LNP-3 | 1.82E+08 | 4.12E+06 | 44.0 |
| LNP-4 | 2.65E+07 | 1.91E+06 | 13.9 |
| LNP-9 | 4.75E+07 | 1.60E+06 | 29.6 |
| LNP-10 | 4.03E+06 | 7.96E+05 | 5.1 |

Conclusion: as shown in Table 13 and Figure 9, the lipid nanoparticle composition LNP-3 containing Compound I-1 exhibited the highest fluorescence intensity in lung, i.e., the highest expression level of luciferase protein in lung, superior to those of LNP-4, LNP-9 and LNP-10. The lung/liver ratio of tissue distribution was obtained by dividing the fluorescence intensity of the lung by the fluorescence intensity of the liver. LNP3 exhibited the highest ratio, followed by LNP-9, and they were superior to LNP-4 and LNP-10. That is, LNPs containing Compounds I-1 and I-8 exhibited higher expression levels in lung.

## Claims

1. A lipid nanoparticle composition comprising:
1) a compound of formula I or a salt thereof, or
2) a compound of formula I or a salt thereof, and a compound of formula II or a salt thereof, 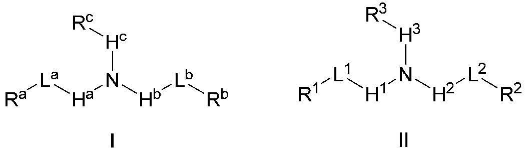 wherein,
L^{a} and L^{b} are each independently selected from the group consisting of -O-, -C(O)-, -OC(O)-, -C(O)O-, -OC(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
H^{a}, H^{b} and H^{c} are each independently selected from the group consisting of C₁₋₁₀ alkylene;
R^{a} and R^{b} are each independently selected from the group consisting of C₁₋₂₄ alkyl and C₂₋₂₄ alkenyl;
R^{c} is selected from the group consisting of NR^{c1}R^{c2} and N⁺R^{c3}R^{c4}R^{c5};
R^{c1} and R^{c2} are each independently selected from the group consisting of hydrogen and C₁₋₆ alkyl;
R^{c3}, R^{c4} and R^{c5} are each independently selected from the group consisting of C₁₋₆ alkyl;
L¹ and L² are each independently selected from the group consisting of -O-, -C(O)-, -OC(O)-, -C(O)O-, -OC(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and at least one of L¹ and L² is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
H¹, H² and H³ are each independently selected from the group consisting of C₁₋₁₀ alkylene;
R¹ and R² are each independently selected from the group consisting of C₁₋₂₄ alkyl and C₂₋₂₄ alkenyl;
R³ is hydroxy.

2. The lipid nanoparticle composition according to claim 1, wherein, L^{a} and L^{b} are each independently selected from the group consisting of -OC(O)-, -C(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-; and L¹ and L² are each independently selected from the group consisting of -OC(O)-, -C(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
preferably, at least one of L^{a} and L^{b} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
further preferably, L^{a} and L^{b} are each independently selected from the group consisting of -OC(O)-, -C(O)O-, -OCH₂C(O)O-, -OC(O)CH₂O-, -OCH₂OC(O)-, -C(O)OCH₂O-, -O(CH₂)₂OC(O)- and -C(O)O(CH₂)₂O-, and at least one of L^{a} and L^{b} is selected from the group consisting of -OCH₂C(O)O-, -OC(O)CH₂O-, -OCH₂OC(O)-, -C(O)OCH₂O-, -O(CH₂)₂OC(O)- or -C(O)O(CH₂)₂O-;
L¹ and L² are each independently selected from the group consisting of -OC(O)-, -C(O)O-, -OCH₂C(O)O-, -OC(O)CH₂O-, -OCH(CH₃)C(O)O-, -OC(O)CH(CH₃)O-, -OC(CH₃)₂C(O)O-, -OC(O)C(CH₃)₂O-, -OCH₂OC(O)-, -C(O)OCH₂O-, -O(CH₂)₂OC(O)-, -C(O)O(CH₂)₂O-, -O(CH₂)₄OC(O)- and -C(O)O(CH₂)₄O-, and at least one of L¹ and L² is selected from the group consisting of -OCH₂C(O)O-, -OC(O)CH₂O-, -OCH(CH₃)C(O)O-, -OC(O)CH(CH₃)O-, -OC(CH₃)₂C(O)O-, -OC(O)C(CH₃)₂O-, -OCH₂OC(O)-, -C(O)OCH₂O-, -O(CH₂)₂OC(O)-, -C(O)O(CH₂)₂O-, -O(CH₂)₄OC(O)- and -C(O)O(CH₂)₄O-.

3. The lipid nanoparticle composition according to claim 1 or 2, wherein, H^{a} is selected from the group consisting of straight or branched C₁₋₁₀ alkylene; H^{b} is selected from the group consisting of straight or branched C₁₋₁₀ alkylene; H^{c} is selected from the group consisting of straight or branched C₁₋₁₀ alkylene; H¹ is selected from the group consisting of straight or branched C₁₋₁₀ alkylene; H² is selected from the group consisting of straight or branched C₁₋₁₀ alkylene; H³ is selected from the group consisting of straight or branched C₁₋₁₀ alkylene;
preferably, H^{a} is selected from the group consisting of straight or branched C₃₋₈ alkylene; H^{b} is selected from the group consisting of straight or branched C₃₋₈ alkylene; H^{c} is selected from the group consisting of straight or branched C₁₋₆ alkylene; H¹ is selected from the group consisting of straight or branched C₃₋₈ alkylene; H² is selected from the group consisting of straight or branched C₃₋₈ alkylene; H³ is selected from the group consisting of straight or branched C₁₋₆ alkylene;
more preferably, H^{a} is selected from the group consisting of -(CH₂)₃-, -(CH₂)₄-, -(CH₂)₅-, -(CH₂)₆-, -(CH₂)₇- and -(CH₂)₈-; H^{b} is selected from the group consisting of -(CH₂)₃-, -(CH₂)₄-, -(CH₂)₅-, -(CH₂)₆-, -(CH₂)₇- and -(CH₂)₈-; H^{c} is selected from the group consisting of -CH₂-, -(CH₂)₂-, -(CH₂)₃- and -(CH₂)₄-;
H¹ is selected from the group consisting of -(CH₂)₃-, -(CH₂)₄-, -(CH₂)₅-, -(CH₂)₆-, -(CH₂)₇- and -(CH₂)₈-; H² is selected from the group consisting of -(CH₂)₃-, -(CH₂)₄-, -(CH₂)₅-, -(CH₂)₆-, -(CH₂)₇- and -(CH₂)₈-; H³ is selected from the group consisting of -CH₂-, -(CH₂)₂-, -(CH₂)₃- and -(CH₂)₄-.

4. The lipid nanoparticle composition according to any one of claims 1 to 3, wherein, R^{a} and R^{b} are each independently selected from the group consisting of straight or branched C₁₋₂₄ alkyl; R¹ and R² are each independently selected from the group consisting of straight or branched C₁₋₂₄ alkyl;
preferably, R^{a} and R^{b} are each independently selected from the group consisting of straight or branched C₄₋₁₈ alkyl; R¹ and R² are each independently selected from the group consisting of straight or branched C₄₋₁₈ alkyl;
more preferably, R^{a} is selected from the group consisting of straight C₄₋₁₈ alkyl, and R^{b} is selected from the group consisting of branched C₄₋₁₈ alkyl, or, R^{a} is selected from the group consisting of branched C₄₋₁₈ alkyl, and R^{b} is selected from the group consisting of branched C₄₋₁₈ alkyl;
R¹ is selected from the group consisting of straight C₄₋₁₈ alkyl, and R² is selected from the group consisting of branched C₄₋₁₈ alkyl, or, R¹ is selected from the group consisting of branched C₄₋₁₈ alkyl, and R² is selected from the group consisting of branched C₄₋₁₈ alkyl;
further preferably, R^{a}, R^{b}, R¹ and R² are each independently selected from the group consisting of : 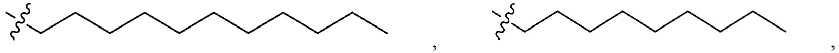 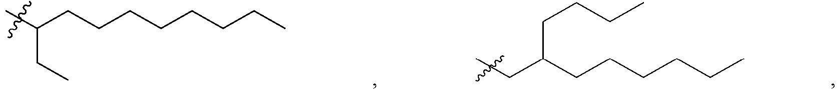 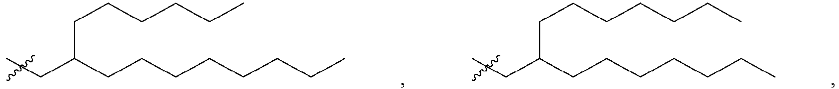 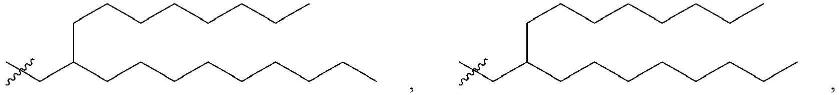 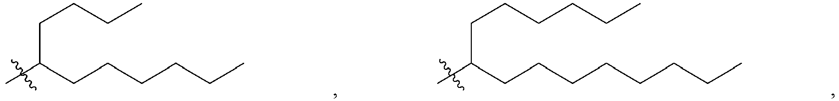 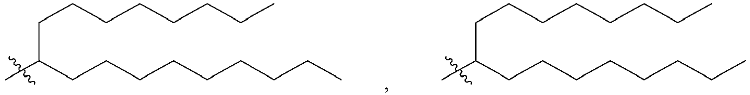 and

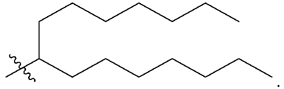

5. The lipid nanoparticle composition according to any one of claims 1 to 4, wherein, the compound of formula I or a salt thereof is a compound of formula Ia or a salt thereof, 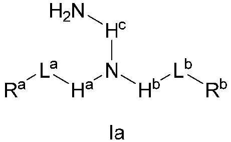 wherein, L^{a}, L^{b}, H^{a}, H^{b}, H^{c}, R^{a} and R^{b} are as defined in any one of claims 1 to 4.

6. The lipid nanoparticle composition according to any one of claims 1 to 5, wherein, the compound of formula I or a salt thereof is selected from the group consisting of compounds of formulas IA, IB, IC, ID, IE and IF or salts thereof, 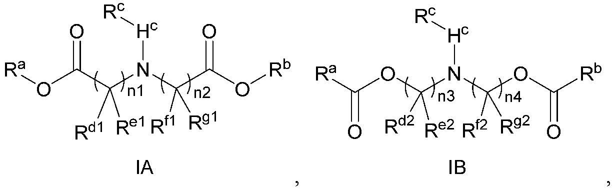 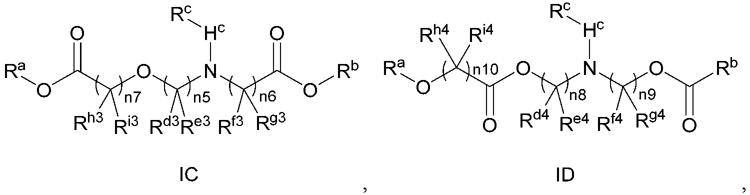 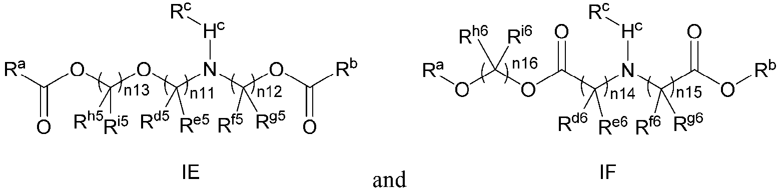
**wherein,** H^{c}, R^{a}, R^{b} and R^{c} are as defined in any one of claims 1 to 5;
R^{d1}, R^{d2}, R^{d3}, R^{d4}, R^{d5}, R^{d6}, R^{e1}, R^{e2}, R^{e3}, R^{e4}, R^{e5}, R^{e6}, R^{f1}, R^{f2}, R^{f3}, R^{f4}, R^{f5}, R^{f6}, R^{g1}, R^{g2}, R^{g3}, R^{g4}, R^{g5}, R^{g6}, R^{h3}, R^{h4}, R^{h5}, R^{h6}, Rⁱ³, Rⁱ⁴, Rⁱ⁵ and Rⁱ⁶ are each independently selected from the group consisting of hydrogen and C₁₋₆ alkyl;
n1, n2, n3, n4, n5, n6, n8, n9, n11, n12, n14 and n15 are each independently selected from the group consisting of 1, 2, 3, 4, 5, 6, 7, 8, 9 and 10;
n7, n10, n13 and n16 are each independently selected from the group consisting of 0, 1, 2, 3, 4, 5 and 6.

7. The lipid nanoparticle composition according to any one of claims 1 to 6, wherein, the compound of formula I is selected from the group consisting of the following structures, 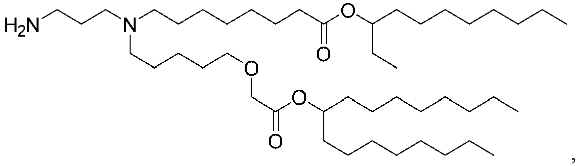 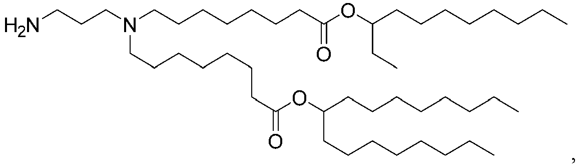 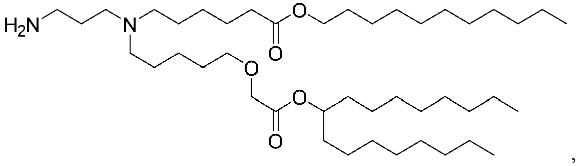 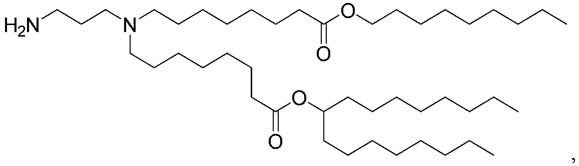 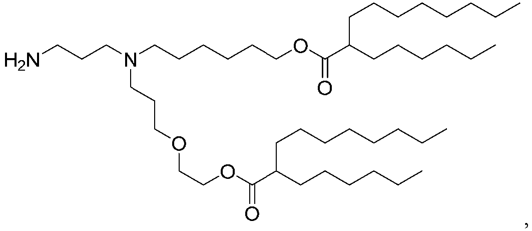 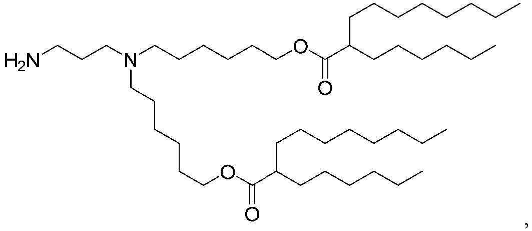 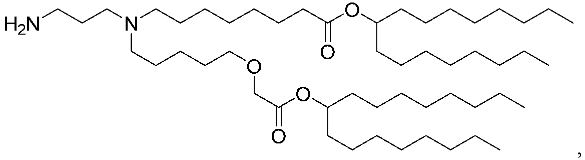 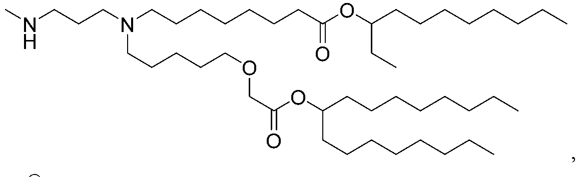 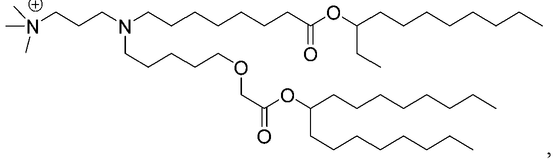 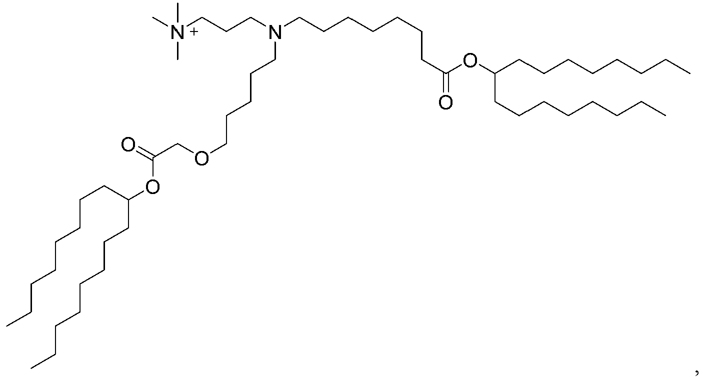 and/or, the compound of formula II is Compound II-1,

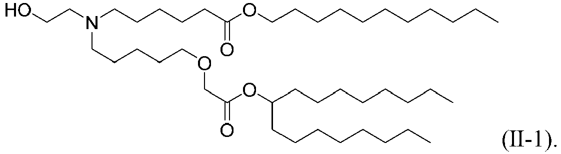

8. The lipid nanoparticle composition according to any one of claims 1 to 7, wherein, the molar percentage of the compound of formula I or a salt thereof to the lipid nanoparticle composition ranges from 10% to 80%, preferably from 10% to 60%;
and/or, the molar percentage of the compound of formula II or a salt thereof to the lipid nanoparticle composition ranges from 5% to 50%, preferably from 10% to 45%.

9. A lipid nanoparticle composition comprising:
1) a primary amine lipid; or
2) a primary amine lipid, and the compound of formula II or a salt thereof according to any one of claims 1 to 7, 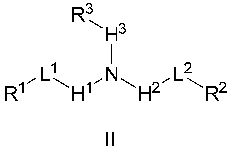
wherein, L¹, L², H¹, H², H³, R¹, R² and R³ are as defined in any one of claims 1 to 4;
preferably, the primary amine lipid is the compound of formula Ia or a salt thereof according to claim 5, 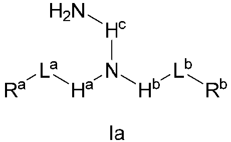
wherein, L^{a}, L^{b}, H^{a}, H^{b}, H^{c}, R^{a} and R^{b} are as defined in any one of claims 1 to 4.

10. The lipid nanoparticle composition according to claim 9, wherein, the molar percentage of the primary amine lipid to the lipid nanoparticle composition ranges from 10% to 80%, preferably from 10% to 60%;
and/or, the molar percentage of the compound of formula II or a salt thereof to the lipid nanoparticle composition ranges from 5% to 50%, preferably from 10% to 45%.

11. The lipid nanoparticle composition according to any one of claims 1 to 10, wherein, the lipid nanoparticle composition comprises a phospholipid;
preferably, the phospholipid is selected from the group consisting of DGTS, DOPE and DSPC, and/or, the molar percentage of the phospholipid to the lipid nanoparticle composition ranges from 1% to 25%;
more preferably, the phospholipid is DSPC, and/or, the molar percentage of the phospholipid to the lipid nanoparticle composition ranges from 1% to 15%.

12. The lipid nanoparticle composition according to any one of claims 1 to 11, wherein, the lipid nanoparticle composition further comprises a steroid;
preferably, the steroid is cholesterol, and/or, the molar percentage of the steroid to the lipid nanoparticle composition ranges from 10% to 50%;
more preferably, the molar percentage of the steroid to the lipid nanoparticle composition ranges from 10% to 45%.

13. The lipid nanoparticle composition according to any one of claims 1 to 12, wherein, the lipid nanoparticle composition further comprises a pegylated lipid;
preferably, the pegylated lipid is a pegylated diacylglycerol, and/or, the molar percentage of the pegylated lipid to the lipid nanoparticle composition ranges from 0.5% to 10%;
more preferably, the pegylated lipid is DMG-PEG, and/or, the molar percentage of the pegylated lipid to the lipid nanoparticle composition ranges from 0.5% to 5%.

14. The lipid nanoparticle composition according to any one of claims 1 to 13, wherein, the composition preferentially delivers a therapeutic agent to a target organ selected from the group consisting of lung, heart, liver, spleen, and stomach;
preferably, the target organ is lung.

15. The lipid nanoparticle composition according to any one of claims 1 to 14, further comprising a therapeutic agent selected from the group consisting of a small molecule, a protein, and a nucleic acid;
preferably, the therapeutic agent is a nucleic acid;
more preferably, the nucleic acid is an mRNA.

16. A compound of formula III or a salt thereof, 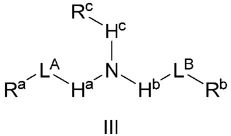 wherein,
L^{A} and L^{B} are each independently selected from the group consisting of -O-, -C(O)-, -OC(O)-, -C(O)O-, -OC(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, and at least one of L^{A} and L^{B} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, wherein C₁₋₆ alkylene is not n-propylidene;
H^{a}, H^{b}, H^{c}, R^{a}, R^{b} and R^{c} are as defined in any one of claims 1 to 7.

17. The compound or a salt thereof according to claim 16, wherein, L^{A} and L^{B} are each independently selected from the group consisting of -OC(O)-, -C(O)O-, -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-, wherein C₁₋₆ alkylene is not n-propylidene;
preferably, at least one of L^{A} and L^{B} is selected from the group consisting of -O-C₁₋₆ alkylene-C(O)O-, -OC(O)-C₁₋₆ alkylene-O-, -O-C₁₋₆ alkylene-OC(O)- and -C(O)O-C₁₋₆ alkylene-O-;
more preferably, L^{A} and L^{B} are each independently selected from the group consisting of -OC(O)-, -C(O)O-, -OCH₂C(O)O-, -OC(O)CH₂O-, -OCH₂OC(O)-, -C(O)OCH₂O-, -O(CH₂)₂OC(O)- and -C(O)O(CH₂)₂O-, and at least one of L^{A} and L^{B} is selected from the group consisting of -OCH₂C(O)O-, -OC(O)CH₂O-, -OCH₂OC(O)-, -C(O)OCH₂O-, -O(CH₂)₂OC(O)- and -C(O)O(CH₂)₂O-.

18. The compound or a salt thereof according to claim 16 or 17, wherein, the compound of formula III or a salt thereof is selected from the group consisting of compounds of formulas IIIA, IIIB, IIIC and IIID or salts thereof, 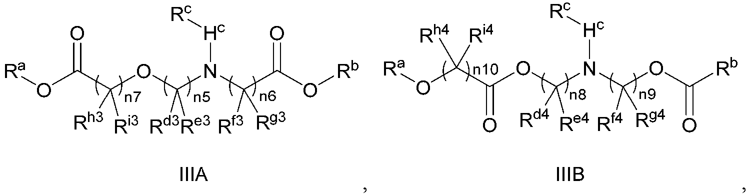 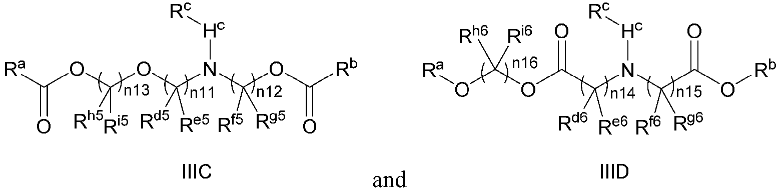
wherein, H^{c}, R^{a}, R^{b} and R^{c} are as defined in any one of claims 1 to 7;
R^{d3}, R^{d4}, R^{d5}, R^{d6}, R^{e3}, R^{e4}, R^{e5}, R^{e6}, R^{f3}, R^{f4}, R^{f5}, R^{f6}, R^{g3}, R^{g4}, R^{g5}, R^{g6}, R^{h3}, R^{h4} R^{h5}, R^{h6}, Rⁱ³, Rⁱ⁴, Rⁱ⁵, Rⁱ⁶ are as defined in claim 6;
n5, n6, n7, n8, n9, n10, n11, n12, n13, n14, n15 and n16 are as defined in claim 6;
preferably, the compound of formula III or a salt thereof is a compound with the following structures or a salt thereof,

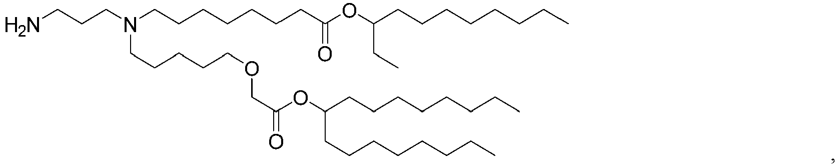

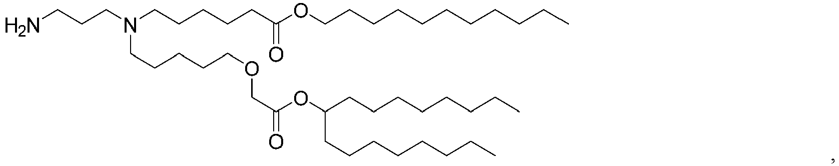

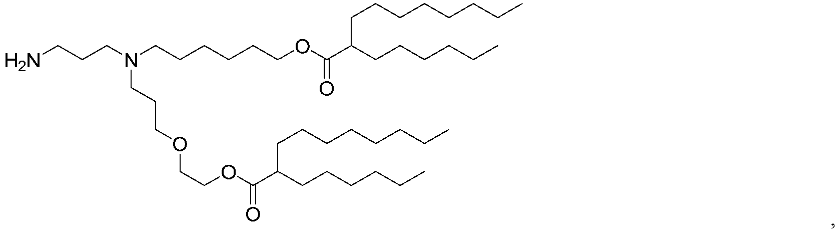

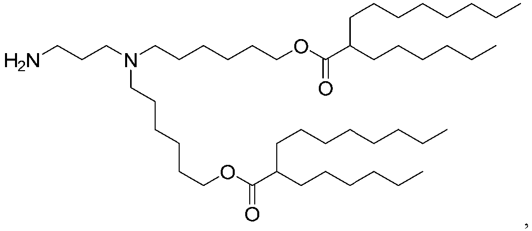

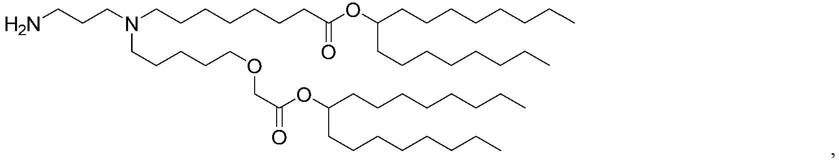

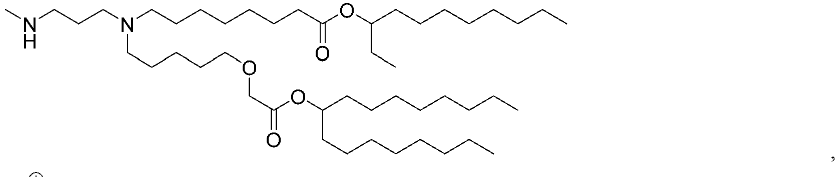

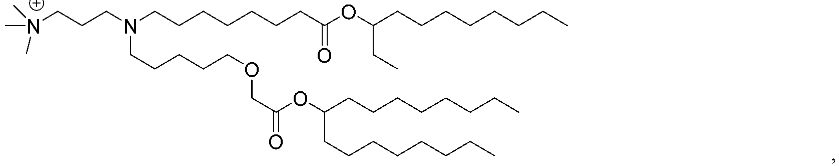

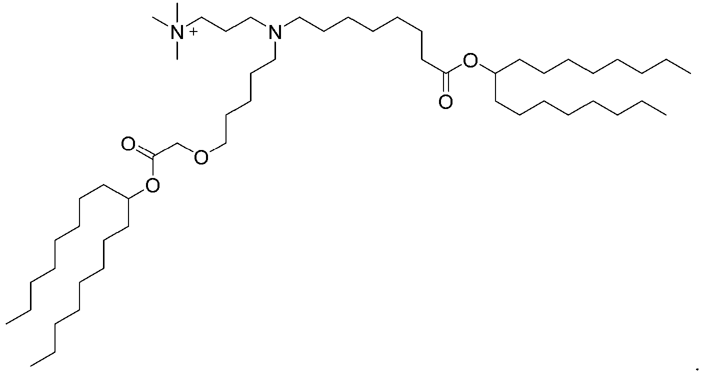

19. A pharmaceutical composition comprising the lipid nanoparticle composition according to any one of claims 1 to 15 or the compound or a salt thereof according to any one of claims 16 to 18, and a pharmaceutically acceptable excipient.

20. Use of the lipid nanoparticle composition according to any one of claims 1 to 15 or the compound or a salt thereof according to any one of claims 16 to 18 or the pharmaceutical composition according to claim 19 in the preparation of a medicament for preventing and/or treating cancer, infection, autoimmune disease, neurodegenerative disease, and inflammation.
